(19) Europäisches Patentamt
European Patent Office
Office européen des brevets

(11) EP 1 796 688 B1

(12) # EUROPEAN PATENT SPECIFICATION

(45) Date of publication and mention of the grant of the patent:
**18.05.2011 Bulletin 2011/20**

(21) Application number: **05794315.1**

(22) Date of filing: **02.09.2005**

(51) Int Cl.:
***A61K 31/704*** *(2006.01)* *A61P 35/00* *(2006.01)*

(86) International application number:
**PCT/US2005/031331**

(87) International publication number:
**WO 2006/028969 (16.03.2006 Gazette 2006/11)**

(54) **PANCREATIC CANCER TREATMENT USING NA+/K+ ATPASE INHIBITORS**

PANKREASKREBSBEHANDLUNG MIT NA+/K+-ATPASE-HEMMERN

TRAITEMENT DU CANCER DU PANCREAS AU MOYEN D'INHIBITEURS D'APTASE NA+/K+

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.09.2004 US 606684 P**

(43) Date of publication of application:
**20.06.2007 Bulletin 2007/25**

(73) Proprietor: **Bionaut Pharmaceuticals Inc**
**Stoneham, MA 02180 (US)**

(72) Inventors:
- **KHODADOUST, Mehran - c/o BT Intern. Ltd.**
**London EC4M 7SB (GB)**
- **SHARMA, Ajay**
**Sudbury , MA 01776 (US)**

(74) Representative: **Tomkinson, Alexandra et al**
**Bailey Walsh & Co**
**5 York Place**
**Leeds LS1 2SD (GB)**

(56) References cited:
**WO-A-00/45165**
**WO-A-03/099011**
**WO-A-2005/060951**
**WO-A-2005/077925**
**US-A1- 2005 182 105**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 796 688 B1

## Description

BACKGROUND OF THE INVENTION

The pancreas can be divided into two parts, the exocrine and endocrine pancreas. Each has a different function. The exocrine pancreas produces various pancreatic enzymes that help break down and digest food. The endocrine pancreas produces hormones (such as insulin) that regulate how the body stores and uses food. About 95% of pancreatic cancers begin in the exocrine pancreas. The rest are cancers of the endocrine pancreas, which are also called islet cell cancers.

According to the National Pancreas Foundation, pancreatic cancer is 4th most common cause of all cancer deaths and the 10th most common malignancy in the United States. Conventional medicine's inability to effectively treat pancreatic cancer is evidenced by survival rates of only 18% at 1 year and 4% at 5 years-one of the poorest 5-year survival rates of any cancer. Pancreatic cancer results in the death of more than 90% of afflicted patients within 12 months. In 2002, about 28,000 Americans died from cancer of the pancreas, The disease is not only common, but it is also extremely difficult to treat. For these and other reasons, lancer of the pancreas has been called "the challenge of the twenty-first century."

Surgical removal ("resection") of the cancer is at present the only chance for a cure for patients with cancer of the pancreas. However, only some 10-15% of all pancreatic cancer cases are eligible for complete surgical removal of the tumor. The surgical resection of most pancreas cancers is called a "Whipple procedure" or "pancreaticoduodenectomy." Although great strides have been made in the surgical treatment of this disease, these operations are very complex, and unless performed by surgeons specially trained and experienced in this procedure, they can be associated with very high rates of operative morbidity and mortality. In general, Whipple resection is a high-risk procedure with a mortality rate of about 15%, and a 5-year survival rate of only 10% (Snady et al. 2000). The 5-year survival rate for patients who do not receive treatment is only about 3%, while for patients underwent a Whipple procedure for cancer of the pancreas is now approaching 25% in best case scenario.

Unfortunately, many cancers of the pancreas are not resectable at the time of presentation. The median survival time for inoperable cases (the majority) is often only a few months. Management of these cases is often based on relieving symptoms (often referred to as palliative care). Chemotherapy and radiation therapy are the main treatments offered to patients whose entire tumor cannot be removed surgically ("unresectable cancers"). In addition, various chemotherapy drugs (one drug or a combination of several drugs) may be used before surgery or following surgery. Often, chemotherapy combined with radiotherapy is used in the conventional treatment of pancreatic cancer (Klinkenbijl et al. 1999; Snady et al. 2000).

Radiation therapy alone can improve pain and may prolong survival. The results are dose-related. Precision external-beam techniques are required. A radiation procedure known as IMRT (intensity modulated radiation therapy) combined with concurrent 5-fluoruracil (5-FU) chemotherapy can provide a definite palliative benefit (symptom relief) with tolerable acute radiation related toxicity for patients with advanced pancreatic cancer (Bai et al. 2003).

In a preliminary report, five patients diagnosed with locally advanced nonresectable pancreatic cancer achieved improved quality of life, delay of local progression, and reduction of biomarker CA19-9 after infusion of colloidal phosphorus 32 ($^{32}P$) and administration of combined chemoradiotherapy. All five of these patients demonstrated cessation of local tumor growth or regression of disease on CT scans for a minimum of 10 months from completion of therapy. Three of these patients survived without local disease progression over 24 months from initiation of therapy, with one patient approaching 36 months. CA19-9 values for all patients declined within weeks after completion of therapy. This new method of isotope delivery has resulted in reduction of tumor volume, normalization of the biomarker CA19-9, and improved performance status in those patients who have localized nonresectable disease without dissemination (cancer spread) (DeNittis et al. 1999).

The chemotherapeutic agent most commonly used to treat cancer of the pancreas is GEMZAR® (Gemcitabin). GEMZAR® works by interfering with cell division and the repair functions, thus preventing the further growth of cancer cells and leading to cell death.

Clinical studies showed that GEMZAR® helped improving survival for some patients with cancer of the pancreas. For example, in a study of GEMZAR® versus the drug 5-FU in previously untreated patients, nearly 1 in 5 patients was alive at 1 year after starting therapy with GEMZAR®, compared with 1 in 50 who were given 5-FU. The typical patient survived about 6 months after starting therapy with GEMZAR®, which was 6 weeks longer than those given 5-FU.

In a study of GEMZAR® in patients previously treated with the drug 5-FU, after starting on GEMZAR®, about 1 in 25 patients was alive at 1 year. After starting on GEMZAR®, the typical patient lived for 4 months. Nearly 1 in 4 patients had improvement in 1 or more of the following for at least 1 month, without any sustained worsening in any of the other symptoms. So far, GEMZAR® is indicated for the treatment of locally advanced or metastatic pancreatic cancer. In treating pancreatic cancer, GEMZAR® is usually given alone, not in combination with other chemotherapy drugs.

It is an object of the present invention to improve the use of those anti-cancer agents, such as GEMZAR®, for

novel and/or more effective approach to treat prancreatic cancer.

SUMMARY OF THE INVENTION

A salient feature of the present invention is the discovery that $Na^+/K^+$-ATPase inhibitors, such as cardiac glycosides (e.g., ouabain and proscillaridin, etc.), can be used either alone or in combination with Standard chemotherapeutic agents or radiotherapy to effectively treat pancreatic cancer.

Once aspect of the invention provides a pharmaceutical formulation comprising a $Na^+/K^+$-ATPase inhibitor in the form of a cardiac glycoside or aglycone form thereof, in combination with an anti-cancer agent, formulated in a pharmaceutically acceptable excipient for the treatment of pancreatic cancer, characterized in that said cardiac glycoside or aglycone form thereof comprises a steroid core with a pyrone substituent at C17 (the "bufadienolides form").

Another aspect of the invention provides a kit for treating a patient having (pancreatic cancer, comprising $Na^+/K^+$-ATPase inhibitor in the form of a cardiac glycoside or aglycone form thereof, in combination with an anti-cancer agent, each formulated in premeasured doses for administration to the patient, characterized in that said cardiac glycoside or aglycone form thereof comprises a steroid core with a pyrone substituent at C17 (the "bufadienolides form"),

Yet another aspect of the invention provides use of a $Na^+/K^+$-ATPase inhibitor in the form of a cardiac glycoside or aglycone form thereof and an anti-cancer agent in the manufacture of a medicament for the treatment of pancreatic cancer, characterized in that said cardiac glycoside or aglycone form thereof comprises a steroid core with a pyrone substituent at C17 (the "bufadienolides form").

Also disclosed herein is a method for promoting treatment of patients having pancreatic cancer, comprising packaging, labelling and/or marketing a $Na^-/K^+$-ATPase inhibitor (e.g. cardiac glycoside) in combination with an anti-cancer agent, to be used in therapy for treating a patient having pancreatic cancer.

Further disclosed herein is a method for promoting treatment of patients having pancreatic cancer, comprising packaging, labelling and/or marketing an anti-cancer agent to be used in conjoint therapy with a $Na^+/K^+$-ATPase inhibitor (e.g. cardiac glycoside) for treating a patient having pancreatic cancer.

In certain embodiments, the cardiac glycoside, in combination with the anti-cancer, has an $IC_{5o}$ for killing one or more different pancreatic cancer cell lines that is at least 2 fold less relative to the $IC_{50}$ of the cardiac glycoside alone, and even more preferably at least 5, 10, 50 or even 100 fold less.

In certain embodiments, the cardiac glycoside, in combination with the anti-cancer agent, has an $EC_{50}$ for treating the neoplastic disorder that is at least 2 fold less relative to the $EC_{50}$ of the cardiac glycoside alone, and even more preferably at least 5, 10, 50 or even 100 fold less.

In certain embodiments, the cardiac glycoside has an $IC_{50}$ for killing one or more different pancreatic cancer cell lines of 500 nM or less, and even more preferably 200 nM, 100 nM, 10 nM or even 1 nM or less.

In certain embodiments, the cardiac glycoside is represented by the general formula:

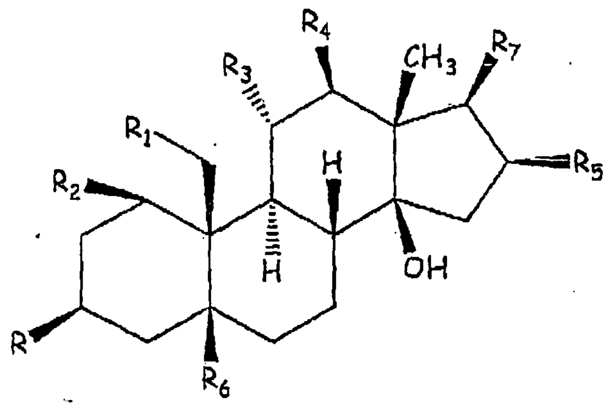

wherein

R represents a glycoside of 1 to 6 sugar residues;

$R_1$ represents hydrogen, -OH or =O;

$R_2$, $R_3$, $R_4$, $R_5$, and $R_6$ each independently represents hydrogen or -OH;

$R_7$ represents

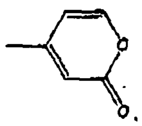

In certain preferred embodiments, the sugar residues are selected from L-rhamnose, D-glucose, D-digitoxose, D-digitalose, D-digginose, D-sarmentose, L-vallarose, and D-fructose. In certain embodiments, these sugars are in the β-conformation. The sugar residues may be acetylated, e.g., to effect the lipophilic character and the kinetics of the entire glycoside. In certain preferred embodiments, the glycoside is 1-4 sugar residues in length.

In certain embodiments, the cardiac glycoside is selected from digitoxigenin, digoxin, lanatoside C, Strophantin K, uzarigenin, desacetyllanatoside A, actyl digitoxin, desacetyllanatoside C, strophanthoside, scillaren A, proscillaridin A, digitoxose, gitoxin, strophanthidiol, oleandrin, acovenoside A, strophanthidine digilanobioside, strophanthidin-d-cymaroside, digitoxigenin-L-rhamnoside, digitoxigenin theretoside, strophanthidin, digoxigenin 3,12-diacetate, gitoxigenin, gitoxigenin 3-acetate, gitoxigenin 3,16-diacetate, 16-acetyl gitoxigenin, acetyl strophanthidin, ouabagenin, 3-epigoxigenin, neriifolin, acetylneriifolin cerberin, theventin, somalin, odoroside, honghelin, desacetyl digilanide, calotropin, calotoxin, convallatoxin, oleandrigenin, bufalin, periplocyrnarin, digoxin (CP 4072), strophanthidin oxime, strophanthidin semicarbazone, strophanthidinic acid lactone acetate, ernicyrnarin, sannentoside D, sarverogenin, sarmentoside A, sarmentogenin, or a pharmaceutically acceptable salt, ester, amide, or prodrug thereof

In certain preferred embodiments, the cardiac glycoside is ouabain or proscillaridin.

Other $Na^+/K^+$-ATPase inhibitors are available in the literature. See, for example, U.S. Patent 5,240,714 which describes a non-digoxin-like $Na^+/K^+$-ATPase inhibitory factor. Recent evidence suggests the existence of several endogenous $Na^+/K^+$-ATPase inhibitors in mammals and animals. For instance, marinobufagenin (3,5-dihydroxy-14,15-epoxy bufodienolide) may be useful in the current combinatorial therapies.

Those skilled in the art can also rely on screening assays to identify compounds that have $Na^+/K^+$-ATPase inhibitory activity. PCT Publications WO00/44931 and WO02/42842 for example, teach high-throughput screening assays for modulators of $Na^+/K^+$-ATPases.

The $Na^+/K^+$-ATPase consists of at least two dissimilar subunits, the large α subunit with all known catalytic functions and the smaller glycosylated β subunit with chaperonic function. In addition there may be a small regulatory, so-called FXYD -peptide. Four α peptide isoforms are known and isoform-specific differences in ATP, $Na^+$ and $K^+$ affinities and in $Ca^{2+}$ sensitivity have been described. Thus changes in $Na^+/K^+$-ATPase isoform distribution in different tissues, as a function of age and development, electrolytes, hormonal conditions etc. may have important physiological implications. Cardiac glycosides like ouabain are specific inhibitors of the $Na^+/K^+$-ATPase. The four α peptide isoforms have similar high ouabain affinities with $K_d$ of around 1 nM or less in almost all mammalian species. In certain embodiments, the $Na^+/K^+$-ATPase inhibitor is more selective for complexes expressed in non-cardiac tissue, relative to cardiac tissue.

In certain embodiments, the anti-cancer agent induces redox-sensitive transcription.

In certain embodiments, the anti-cancer agent induces HIF-1α-dependent transcription.

In certain embodiments, the anti-cancer agent induces expression of one or more of cyclin G2, IGF2, IGF-BP1, IGF-BP2, IGF-BP3, EGF, WAF-1, TGF-α, TGF-β3, ADM, EPO, IGF2, EG-VEGF, VEGF, NOS2, LEP, LRP1, HK1, HK2, AMF/GP1, ENO1, GLUT1, GAPDH, LDHA, PFKBF3, PKFL, MIC1, NIP3, NIX and/or RTP801.

In certain embodiments, the anti-cancer agent induces mitochondrial dysfunction and/or caspase activation.

In certain embodiments, the anti-cancer agent induces cell cycle arrest at G2/M in the absence of said cardiac glycoside.

In certain embodiments, the anti-cancer agent is an inhibitor of chromatin function.

In certain embodiments, the anti-cancer agent is a DNA topoisomerase inhibitor, such as selected from adriamycin, amsacrine, camptothecin, daunorubicin, dactinomycin, doxorubicin, eniposide, epirubicin, etoposide, idarubicin, irinotecan (CPT-11) and mitoxantrone.

In certain embodiments, the anti-cancer agent is a microtubule inhibiting drug, such as a taxane, including paclitaxel, docetaxel, vincristin, vinblastin, nocodazole, epothilones and navelbine.

In certain embodiments, the anti-cancer agent is a DNA damaging agent, such as actinomycin, amsacrine, anthracyclines, bleomycin, busulfan, camptothecin, carboplatin, chlorambucil, cisplatin, cyclophosphamide, cytoxan, dactinomycin, daunorubicin, docetaxel, doxorubicin, epirubicin, hexamethylmelamineoxaliplatin, iphosphamide, melphalan, merchlorehtamine, mitomycin, mitoxantrone, nitrosourea, plicamycin, procarbazine, taxol, taxotere, teniposide, triethylenethiophosphoramide and etoposide (VP16).

In certain embodiments, the anti-cancer agent is an antimetabolite, such as a folate antagonists, or a nucleoside analog. Exemplary nucleoside analogs include pyrimidine analogs, such as 5-fluorouracil; cytosine arabinoside, and azacitidine. In other embodiments, the nucleoside analog is a purine analog, such as 6-mercaptopurine; azathioprine; 5-iodo-2'-deoxyuridine; 6-thioguanine; 2-deoxycoformycin, cladribine, cytarabine, fludarabine, mercaptopurine, thioguanine, and pentostatin. In certain embodiments, the nucleoside analog is selected from AZT (zidovudine); ACV; valacylovir; famiciclovir; acyclovir; cidofovir; penciclovir; ganciclovir; Ribavirin; ddC; ddl (zalcitabine); lamuvidine; Abacavir; Adefovir; Didanosine; d4T (stavudine); 3TC; BW 1592; PMEA/bis-POM PMEA; ddT, HPMPC, HPMPG, HPMPA, PMEA, PMEG, dOTC; DAPD; Ara-AC, pentostatin; dihydro-5-azacytidine; tiazofurin; sangivamycin; Ara-A (vidarabine); 6-MMPR; 5-FUDR (floxuridine); cytarabine (Ara-C; cytosine arabinoside); 5-azacytidine (azacitidine); HBG [9-(4-hydroxybutyl)guanine], (1S,4R)-4-[2-amino-6-cyclopropyl-amino)-9H-purin-9-yl]-2-cyclopentene-1-methanol succinate ("159U89"), uridine; thymidine; idoxuridine; 3-deazauridine; cyclocytidine; dihydro-5-azacytidine; triciribine, ribavirin, and fludrabine.

In certain embodiments, the nucleoside analog is a phosphate ester selected from the group consisting of: Acyclovir; 1-β-D-arabinofuranosyl-E-5-(2-bromovinyl)uracil; 2'-fluorocarbocyclic-2'-deoxyguanosine; 6'-fluorocarbocyclic-2' deoxyguanosine; 1-(β-D-arabinofuranosyl)-5(E)-(2-iodovinyl)uracil; {(1r-1α, 2β, 3α)-2-amino-9-(2,3-bis(hydroxymethyl)cyclobutyl)-6H-purin-6-one}Lobucavir; 9H-purin-2-amine, 9-((2-(1-methylethoxy)-1-((1-methylethoxy)methyl)ethoxy)methyl)-(9Cl); trifluorothymidine; 9->(1,3-dihydroxy-2-propoxy)methylguanine (ganciclovir); 5-ethyl-2'-deoxyuridine; E-5-(2-bromovinyl)-2'-deoxyuridine; 5-(2-chloroethyl)-2'-deoxyuridine; buciclovir; 6-deoxyacyclovir; 9-(4-hydroxy-3-hydroxymethylbut-1-yl)guanine; E-5-(2-iodovinyl)-2'-deoxyuridine; 5-vinyl-1-β-D-arabinofuranosyluracil; 1-β-D-arabinofuranosylthymine; 2'-nor-2'deoxyguanosine; and 1-β-D-arabinofuranosyladenine.

In certain embodiments, the nucleoside analog modulates intracellular CTP and/or dCTP metabolism.

In certain preferred embodiments, the nucleoside analog is gemcitabine (GEMZAR®).

In certain embodiments, the anti-cancer agent is a DNA synthesis inhibitor, such as a thymidilate synthase inhibitors (such as 5-fluorouracil), a dihydrofolate reductase inhibitor (such as methoxtrexate), or a DNA polymerase inhibitor (such as fludarabine).

In certain embodiments, the anti-cancer agent is a DNA binding agent, such as an intercalating agent.

In certain embodiments, the anti-cancer agent is a DNA repair inhibitor.

In certain embodiments, the anti-cancer agent is part of a combinatorial therapy selected from ABV, ABVD, AC (Breast), AC (Sarcoma), AC (Neuroblastoma), ACE, ACe, AD, AP, ARAC-DNR, B-CAVe, BCVPP, BEACOPP, BEP, BIP, BOMP, CA, CABO, CAF, CAL-G, CAMP, CAP, CaT, CAV, CAVE ADD, CA-VP16, CC, CDDP/VP-16, CEF, CEPP (B), CEV, CF, CHAP, ChlVPP, CHOP, CHOP-BLEO, CISCA, CLD-BOMP, CMF, CMFP, CMFVP, CMV, CNF, CNOP, COB, CODE, COMLA, COMP, Cooper Regimen, COP, COPE, COPP, CP - Chronic Lymphocytic Leukemia, CP - Ovarian Cancer, CT, CVD, CVI, CVP, CVPP, CYVADIC, DA, DAT, DAV, DCT, DHAP, DI, DTIC/Tamoxifen, DVP, EAP, EC, EFP, ELF, EMA 86, EP, EVA, FAC, FAM, FAMTX, FAP, F-CL, FEC, FED, FL, FZ, HDMTX, Hexa-CAF, ICE-T, IDMTX/6-MP, IE, IfoVP, IPA, M-2, MAC-III, MACC, MACOP-B, MAID, m-BACOD, MBC, MC, MF, MICE, MINE, mini-BEAM, MOBP, MOP, MOPP, MOPP/ABV, MP - multiple myeloma, MP- prostate cancer, MTX/6-MO, MTX/6-MP/VP, MTX-CDDPAdr, MV - breast cancer, MV - acute myelocytic leukemia, M-VAC Methotrexate, MVP Mitomycin, MVPP, NFL, NOVP, OPA, OPPA, PAC, PAC-I, PA-CI, PC, PCV, PE, PFL, POC, ProMACE, ProMACE/cytaBOM, PRoMACE/MOPP, Pt/VM, PVA, PVB, PVDA, SMF, TAD, TCF, TIP, TTT, Topo/CTX, VAB-6, VAC, VACAdr, VAD, VATH, VBAP, VBCMP, VC, VCAP, VD, VelP, VIP, VM, VMCP, VP, V-TAD, 5 + 2, 7 + 3, "8 in 1".

In certain embodiments, the anti-cancer agent is selected from altretamine, aminoglutethimide, amsacrine, anastrozole, asparaginase, bcg, bicalutamide, bleomycin, buserelin, busulfan, calcium folinate, campothecin, capecitabine, carboplatin, carmustine, chlorambucil, cisplatin, cladribine, clodronate, colchicine, crisantaspase, cyclophosphamide, cyproterone, cytarabine, dacarbazine, dactinomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, ironotecan, letrozole, leucovorin, leuprolide, levamisole, lomustine, mechlorethamine, medroxyprogesterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.

In certain embodiments, the anti-cancer agent is selected from tamoxifen, 4-(3-chloro-4-fluorophenylamino)-7-methoxy-6-(3-(4-α-morpholinyl)propoxy)quinazoline, 4-(3-ethynylphenylamino)-6,7-bis(2-methoxyethoxy)quinazoline, hormones, steroids, steroid synthetic analogs, 17a-ethinylestradiol, diethylstilbestrol, testosterone, prednisone, fluoxymesterone, dromostanolone propionate, testolactone, megestrolacetate, methylprednisolone, methyl-testosterone, prednisolone, triamcinolone, chlorotrianisene, hydroxyprogesterone, aminoglutethimide, estramustine, medroxyprogesteroneacetate, leuprolide, flutamide, toremifene, Zoladex, antiangiogenics, matrix metalloproteinase inhibitors, VEGF inhibitors, ZD6474, SU6668, SU11248, anti-Her-2 antibodies (ZD1839 and OSI774), EGFR inhibitors, EKB-569, Imclone antibody C225, src inhibitors, bicalutamide, epidermal growth factor inhibitors, Her-2 inhibitors, MEK-1 kinase

inhibitors, MAPK kinase inhibitors, P13 inhibitors, PDGF inhibitors, combretastatins, MET kinase inhibitors, MAP kinase inhibitors, inhibitors of non-receptor and receptor tyrosine kinases (imatinib), inhibitors of integrin signaling, and inhibitors of insulin-like growth factor receptors.

In certain embodiments, the subject cardiac glycosides or combinations with anti-cancer agents are used to inhibit growth of a metastasized pancreatic tumor in an organ selected from: lung, prostate, breast, colon, liver, brain, kidney, skin, ovary, and blood.

It is contemplated that all embodiments of the invention may be combined with any other embodiment(s) of the invention.

## BRIEF DESCRIPTION OF THE DRAWINGS

Figure 1. Schematic diagram of using Sentinel Line promoter-less trap vectors to generate active genetic sites expressing drug selection markers and/or reporters.

Figure 2. Schematic diagram of creating a Sentinel Line by sequential isolation of cells resistant to positive and negative selection drugs.

Figure 3. FACS Analysis of Sentinel Lines. Sentinel Lines were developed by transfecting A549 (NSCLC lung cancer) and Panc-1 (pancreatic cancer) cell lines with gene-trap vectors containing *E. coli* LacZ- encoded β-galactosidase (β-gal) as the reporter gene. The β-gal

Figure 4. activity in Sentinel Lines (green) was measured by flow cytometry using a fluorogenic substrate fluoresescein di-beta-D- galactopyranoside (FDG). The autofluorescence of untransfected control cells is shown in purple. The graphs indicate frequency of cells (y-axis) and intensity of fluorescence (x-axis) in log scale. The bar charts on the right depict median fluorescent units of the FACS curves. They indicate a high level of reporter activity at the targeted site. Demonstrates that BNC1 induces ROS production and inhibits HIF-1α induction in tumor cells.

Figure 5. Demonstrates that the cardiac glycoside compounds BNC1 and BNC4 directly or indirectly inhibits in tumor cells the secretion of the angiogenesis factor VEGF.

Figure 6. These four charts show FACS analysis of response of a NSCLC Sentinel Line (A549), when treated 40 hrs with four indicated agents. Control (untreated) is shown in purple. Arrow pointing to the right indicates increase in reporter activity whereas inhibitory effect is indicated by arrow pointing to the left. The results indicate that standard chemotherapy drugs turn on survival response in tumor cells.

Figure 7. Effect of BNC4 on Gemcitabine-induced stress responses visualized by A549 Sentinel Lines™.

Figure 8. Pharmacokinetic analysis of BNC1 delivered by osmotic pumps. Osmotic pumps (Model 2002, Alzet Inc) containing 200 μl of BNC1 at 50, 30 or 20 mg/ml in 50% DMSO were implanted subcutaneously into nude mice. Mice were sacrificed after 24, 48 or 168 hrs, and plasma was extracted and analyzed for BNC1 by LC-MS. The values shown are average of 3 animals per point.

Figure 9. Shows effect of BNC1 alone or in combination with standard chemotherapy on growth of xenografted human pancreatic tumors in nude mice.

Figure 10. Shows anti-tumor activity of BNC1 and Cytoxan against Gaki-1 human renal cancer xenograft.

Figure 11. Shows anti-tumor activity of BNC1 alone or in combination with Carboplatin in A549 human non-small-cell-lung carcinoma.

Figure 12. Titration of BNC1 to determine minimum effective dose effective against Panc-1 human pancreatic xenograft in nude mice. BNC1 (sc, osmotic pumps) was tested at 10, 5 and 2 mg/ml.

Figure 13. Combination of BNC1 with Gemcitabine is more effective than either drug alone against Panc-1 xenografts.

Figure 14. Combination of BNC1 with 5-FU is more effective than either drug alone against Panc-1 xenografts.

Figure 15. Comparison of BNC1 and BNC4 in inhibiting hypoxia-mediated HIF-1α induction in human tumor cells (Caki-1 and Panc-1 cells).

Figure 16. BNC4 blocks HIF-1α induction by a prolyl-hydroxylase inhibitor under normoxia.

## DETAILED DESCRIPTION OF THE INVENTION

### *I. Overview*

The present invention is based in part on the discovery that $Na^+/K^+$-ATPase inhibitors (e.g. cardiac glycosides, such as ouabain and proscillaridin), are potent agents for treating pancreatic cancers when used alone or in combination with other anti-tumor agents. Thus a salient feature of the present invention is the discovery that $Na^+/K^+$-ATPase inhibitors can be used either alone or in combination with these anti-cancer agents to more effectively treat pancreatic cancer.

*II. Definitions*

As used herein the term "animal" refers to mammals, preferably mammals such as humans. Likewise, a "patient" or "subject" to be treated by the method of the invention can mean either a human or non-human animal.
As used herein, the term "pancreatic cancer" refers to any neoplastic disorder, including cellular disorders in pancrease. In a preferred embodiment, a pancreatic cancer originates from pancreatic cells. However, cancers originating from other organs may metastasize to pancrease. In certain embodiments, pancreatic cancers are not treated by any clinical means. In some other embodiments, the pancreatic cancer is one that cannot be treated by surgical reduction. In yet another embodiment, the pancreatic cancer is refractory to treatments by conventional chemo-therapy and/or radio-therapy.
The "growth state" of a cell refers to the rate of proliferation of the cell and the state of differentiation of the cell.
As used herein, "hyperproliferative disease" or "hyperproliferative disorder" refers to any disorder which is caused by or is manifested by unwanted proliferation of cells in a patient.
As used herein, "proliferating" and "proliferation" refer to cells undergoing mitosis.
As used herein, "unwanted proliferation" means cell division and growth that is not part of normal cellular turnover, metabolism, growth, or propagation of the whole organism. Unwanted proliferation of cells is seen in tumors and other pathological proliferation of cells, does not serve normal function, and for the most part will continue unbridled at a growth rate exceeding that of cells of a normal tissue in the absence of outside intervention. A pathological state that ensues because of the unwanted proliferation of cells is referred herein as a "hyperproliferative disease" or "hyperproliferative disorder."
As used herein, "transformed cells" refers to cells that have spontaneously converted to a state of unrestrained growth, i.e., they have acquired the ability to grow through an indefinite number of divisions in culture. Transformed cells may be characterized by such terms as neoplastic, anaplastic and/or hyperplastic, with respect to their loss of growth control. For purposes of this invention, the terms "transformed phenotype of malignant mammalian cells" and "transformed phenotype " are intended to encompass, but not be limited to, any of the following phenotypic traits associated with cellular transformation of mammalian cells: immortalization, morphological or growth transformation, and tumorigenicity, as detected by prolonged growth in cell culture, growth in semi-solid media, or tumorigenic growth in immuno-incompetent or syngeneic animals.

*III. Exemplary Embodiments*

Many $Na^+/K^+$-ATPase inhibitors are available in the literature. See, for example, U.S. Patent 5,240,714 which describes a non-digoxin-like $Na^+/K^+$-ATPase inhibitory factor. Recent evidence suggests the existence of several endogenous $Na^+/K^+$-ATPase inhibitors in mammals and animals. For instance, marinobufagenin (3,5-dihydroxy-14,15-epoxy bufodienolide) may be useful in the current combinatorial therapies.
Those skilled in the art can also rely on screening assays to identify compounds that have $Na^+/K^+$-ATPase inhibitory activity. PCT Publications WO00/44931 and WO02/42842, for example, teach high-throughput screening assays for modulators of $Na^+/K^+$-ATPases.
The $Na^+/K^+$-ATPase consists of at least two dissimilar subunits, the large $\alpha$ subunit with all known catalytic functions and the smaller glycosylated $\beta$ subunit with chaperonic function. In addition there may be a small regulatory, so-called FXYD -peptide. Four $\alpha$ peptide isoforms are known and isoform-specific differences in ATP, $Na^+$ and $K^+$ affinities and in $Ca^{2+}$ sensitivity have been described. Thus changes in $Na^+/K^+$-ATPase isoform distribution in different tissues, as a function of age and development, electrolytes, hormonal conditions etc. may have important physiological implications. Cardiac glycosides like ouabain are specific inhibitors of the $Na^+/K^+$-ATPase. The four $\alpha$ peptide isoforms have similar high ouabain affinities with $K_d$ of around 1 nM or less in almost all mammalian species. In certain embodiments, the $Na^+/K^+$-ATPase inhibitor is more selective for complexes expressed in non-cardiac tissue, relative to cardiac tissue. The following section describes a preferred embodiments of $Na^+/K^+$-ATPase inhibitors - cardiac glycosides.

A. Exemplary Cardiac Glycosides

The inventors have demonstrated that cardiac glycosides, either alone or in combination with other standard anti-cancer chemo- and/or radio-therapeutics, are effective in killing pancreatic cancer cells. The inventors have also observed that cardiac glycosides have potent antiproliferative effects in pancreatic cancer cell lines.
The term "cardiac glycoside" or "cardiac steroid" is used in the medical field to refer to a category of compounds tending to have positive inotropic effects on the heart. As a general class of compounds, cardiac glycosides comprise a steroid core with either a pyrone or butenolide substituent at C17 (the "pyrone form" and "butenolide form"). Additionally, cardiac glycosides may optionally be glycosylated at C3. Most cardiac glycosides include one to four sugars attached to the 3β-OH group. The sugars most commonly used include L-rhamnose, D-glucose, D-digitoxose, D-digitalose, D-digginose, D-sarmentose, L-vallarose, and D-fructose. In general, the sugars affect the pharmacokinetics of a cardiac glycoside with little other effect on biological activity. For this reason, aglycone forms of cardiac glycosides are available and are intended to be encompassed by the term "cardiac glycoside" as used herein. The pharmacokinetics of a cardiac glycoside may be adjusted by adjusting the hydrophobicity of the molecule, with increasing hydrophobicity tending to result in greater absorbtion and an increased half-life. Sugar moieties may be modified with one or more groups, such as an acetyl group.

A large number of cardiac glycosides are known in the art for the purpose of treating cardiovascular disorders. Given the significant number of cardiac glycosides that have proven to have anticancer effects in the assays disclosed herein, it is expected that most or all of the cardiac glycosides used for the treatment of cardiovascular disorders may also be used for treating proliferative disorders. Examples of cardiac glycosides include ouabain, proscillaridin A, digitoxigenin, digoxin and lanatoside C. Additional examples of cardiac glycosides include: Strophantin K, uzarigenin, desacetyllanatoside A, actyl digitoxin, desacetyllanatoside C, strophanthoside, scillaren A, digitoxose, gitoxin, strophanthidiol, oleandrin, acovenoside A, strophanthidine digilanobioside, strophanthidin-d-cymaroside, digitoxigenin-L-rhamnoside, digitoxigenin theretoside, strophanthidin, digoxigenin 3,12-diacetate, gitoxigenin, gitoxigenin 3-acetate, gitoxigenin 3,16-diacetate, 16-acetyl gitoxigenin, acetyl strophanthidin, ouabagenin, 3-epigoxigenin, neriifolin, acetylneriifolin cerberin, theventin, somalin, odoroside, honghelin, desacetyl digilanide, calotropin and calotoxin. Cardiac glycosides may be evaluated for effectiveness in the treatment of cancer by a variety of methods, including, for example: evaluating the killing effects of a cardiac glycoside in a panel of pancreatic cancer cell lines, or evaluating the effects of a cardiac glycoside on pancreatic cancer cell proliferation.

Notably, cardiac glycosides affect proliferation of cancer cell lines at a concentration well below the known toxicity level. The $IC_{50}$ measured for ouabain across several different cancer cell lines ranged from about 15 nM to about 600 nM, or about 80 nM to about 300 nM. The concentration at which a cardiac glycoside is effective as part of an antiproliferative treatment may be further decreased by combination with an additional agent, such as a redox effector or a steroid signal modulator. For example, as shown herein, the concentration at which a cardiac glycoside (e.g. ouabain or proscillaridin) is effective for inhibiting proliferation of pancreatic cancer cells is decreased by at least 2-fold when in combination with sub-optimal level of Gemcitabin (GEMZAR®). Therefore, in certain embodiments, the invention provides combination therapies of cardiac glycosides with, for example, pancreatic cancer drugs such as Gemcitabin (GEMZAR®). Additionally, cardiac glycosides may be combined with radiation therapy, taking advantage of the radiosensitizing effect that many cardiac glycosides have.

B. <u>Exemplary Anti-cancer Agents</u>

Many chemotherapeutic drugs have been evaluated for treating pancreatic cancer, unfortunately, no single chemotherapy drug so far has produced a significant response rate or median survival rate. Therefore, a combination of several drugs such as 5-fluorouracil, streptozotocin, and cisplatin is not uncommon in chemotherapy for pancreatic cancer (Snady et al. 2000). Understandably, any chemotherapy treatment plan must be highly individualized according to the type, location, and progression of each patient's pancreatic cancer. Such anti-cancer agents may all be combined with the subject cardiac glycosides in treating pancreatic cancer. The following is a brief description of several most commonly used chemotherapeutic agents for treating pancreatic cancers. All such therapeutic regimens are suitable for conjoint therapy with the subject cardiac glycosides in treating pancreatic cancer.

**5-Fluorouracil**

Chemotherapy with 5-fluorouracil (5-FU) is associated with a response rate of less than 20% in pancreatic cancer and does not improve the survival rate. As a result of these disappointing findings, multiple drug therapies have been used, but without much greater success.

5-FU combined with ginkgo biloba extract was evaluated in 32 individuals with advanced pancreatic cancer. Progressive disease was observed in 22 (68.8%), no change was observed in seven (21.9%), and partial response was observed in three (9.4%). The overall response was 9.4%. In comparison with studies using the drug gemcitabine, the combination of 5-FU and ginkgo biloba extract showed comparable response rates with a low toxicity. The results suggest a good benefit-risk ratio for the combination of 5-FU and ginkgo biloba extract in the treatment of pancreatic cancer (Hauns et al. 1999).

In Europe, oncologists have combined 5-FU with borage oil (gamma-linolenic acid) to improve absorption of 5-FU (Umejima et al. 1995).

A Phase III trial using chemotherapy combined with radiotherapy and 5-FU found only minor toxicity occurred in patients. Adjuvant radiotherapy in combination with 5-FU was safe and well tolerated. The treated group showed some improvement in survival rates (cancer of the head of the pancreas, 26% in the observation group versus 35% in the treatment group; periampullary cancer, 63% in the observation group versus 67% in the treatment group).

**Accutane**

Based on the need to inhibit pancreatic cancer cell division at different stages of its growth and induce apoptosis (programmed cell death) of cancer cells, multiple therapeutic modalities are often recommended. One successful treatment modality is to combine the differentiating-inducing drug Accutane (13-cis-retinoic acid) with other chemotherapy drugs, such as 5-FU.

A combination of 13-cis-retinoic acid (Accutane) and interferon-alpha was tested in a Phase II trial of 22 patients with pancreatic cancer. One patient experienced partial remission and 14 patients demonstrated stable disease for about 5 months (Brembeck et al. 1998).

**Gemcitabine**

Gemcitabine hydrochloride (GEMZAR®), given by injection, has shown moderate promise. Gemcitabine inhibits the enzyme responsible for DNA synthesis. Treatment with gemcitabine alone achieved clinical benefit in 20-30% of patients; the 1-year survival rate of gemcitabine treated patients was 18% compared with a 2% survival rate for patients treated with a combination of gemcitabine and 5-FU (Heinemann 2001).

Some studies have shown a modest improvement by combining gemcitabine with 5-FU or cisplatin (Brodowicz et al. 2000; Oettle et al. 2000). Pancreatic cancer cells with a mutant K-ras oncogene are more susceptible to the cancer killing effects of gemcitabine. More than 85% of pancreatic cancers expressed a mutated K-ras oncogene (Kijima et al. 2000).

**Ifosfamide**

Twenty-nine patients with pancreatic cancer were treated by injection with Ifosfamide, a chemotherapy drug approved for use in a wide variety of cancers. In addition to Ifosfamide, N-acetylcysteine (NAC) was administered as a protective agent. Nausea and vomiting occurred in the majority of the treated patients. Other adverse effects noted were mild myelosuppression, central nervous system (CNS) toxicity, and one case of acute renal (kidney) failure. One complete response and five partial responses were observed in 27 patients (Loehrer et al. 1985; Einhorn et al. 1986).

**Paclitaxel**

Paclitaxel (Taxol) is a drug extracted from the needles of the European yew *Taxus baccata* that inhibits microtubule syntheses, an essential part of cell division and growth. Taxol was shown to inhibit growth in human pancreatic adenocarcinoma cell lines with mutant p53 genes (Gururajanna et al. 1999). Taxol combined with Tiazofurin had a synergistic effect in human pancreatic, ovarian, and lung carcinoma cell lines (Taniki et al. 1993).

**Docetaxel**

Docetaxel (Taxotere) is a chemical synthesized from *Taxus baccata* that retains the unique mechanism of action of Taxol and inhibits the depolymerization of microtubules into tubulin. Based on the results of Phase II clinical trials, docetaxel is currently approved for use in breast and lung cancer.

Taxotere was shown to be active with 80% complete regressions against advanced C38 colon adenocarcinoma and PO3 pancreatic ductal adenocarcinoma (Lavelle et al. 1993).

In a Phase II study of 40 patients with pancreatic cancer who were treated with docetaxel, six patients (15%) experienced a partial response and 15 patients (38%) experienced stable disease. The median duration of response was 5.1 months, with a range of 3.1-7.2 months (Rougier et al. 2000).

Docetaxel and gemcitabine were used in combination to treat 15 pancreatic cancer patients. Four patients (27%) achieved an objective response as observed by CT scan, including one complete response. Seven patients (47%) had subjective improvement and decreased serum marker levels of CA 19-9. In vitro testing showed that docetaxel and gemcitabine were minimally effective alone, but when combined they displayed additional antiproliferative effects (Sherman et al. 2001).

A second study of 54 patients treated with docetaxel and gemcitabine had similar results: Seven patients (13%) achieved partial response, and 18 (33%) achieved stable disease. The median duration of response was 24 weeks, time to tumor progression was 32 weeks, and overall survival was 26 weeks (Stathopoulos et al. 2001).

**Trimetrexate**

Trimetrexate (Neutrexin) is a folate antagonist structurally similar to methotrexate and trimethoprim. The FDA

approved Trimetrexate in 1993 for use in pancreatic and colorectal cancer. Trimetrexate inhibits the enzyme dihydrofolate reductase, which converts dihydrofolate into the biologically active tetrahydrofolate that is needed for the synthesis of purines, DNA, and cellular proteins.

### Caffeine

As noted earlier, caffeine was once thought to be associated with an increased risk of developing pancreatic cancer, but studies do not provide strong evidence to support an increased risk from drinking coffee, and caffeine has been used in combination with chemotherapy drugs and analgesics (pain-relieving drugs).

A Phase II study using cisplatin, cytarabine, and caffeine with a continuous IV infusion of 5-FU for the treatment of pancreatic carcinoma was carried out on thirty eligible patients. A complete remission was seen in two patients and partial remission was seen in three patients, with an overall response rate of 16.7%. The median survival was 5.0 months (range: 0.3-32.4 months), and 16.7% and 10% of patients were alive at 1 and 2 years, respectively. Although the combination chemotherapy treatment produced durable responses in pancreatic cancer, the toxicity was substantial (Ahmed et al. 2000).

In a Phase I clinical trial, 7 of 18 patients with advanced pancreatic cancer had partial responses to caffeine. A subsequent Phase III clinical trial compared caffeine versus standard treatment using a combination of streptozotocin, mitomycin, and 5-FU (referred to as SMF). Two patients (5.5%) on caffeine treatment and four patients (10.2%) on SMF treatment had objective responses (partial response or improvement). No complete remission was observed. The median duration of survival for all patients on the SMF treatment protocol was 10 months, while median duration of survival was 5 months on the caffeine treatment. Neither regimen was found to be effective treatment for advanced pancreatic cancer (Kelsen et al. 1991).

In a Phase I/II study, 28 patients with advanced pancreatic adenocarcinoma were treated with cisplatin, high-dose cytarabine (ARA-C), and caffeine. Of the 28 patients, 18 had measurable or assessable disease; 7 (39%) had partial responses. The median response duration was 6.2 months. Median survival for responders was 9.5 months, with two patients surviving for more than 18 months. Median survival for all patients was 6.1 months (Dougherty et al. 1989).

Caffeine, injected into male Wistar rats that had been injected with a drug that is known to causes tumors impeded DNA synthesis. A dose-dependant relationship was observed with the higher dose decreasing the total number of nodules (Denda et al. 1983).

In addition, at least about 64 clinical trials for pancreatic cancer were actively underway via the National Institute of Health. For a list of these trials, visit http://clinicaltrials.gov/ct/search?term=pancreatic+cancer or the Cancer Option Web site at www.CancerOption.com. Chemotherapeutic regimens described in these trials are all suitable for conjoint therapy with the subject cardiac glycosides in treating pancreatic cancer. Described below are some of the new drugs for treating pancreatic cancers.

### Camptothecin

Camptothecin is derived from the wood and bark of the Chinese tree *Camptotheca acuminata,* the so-called "happy tree." The active ingredient was discovered in 1966 by the same researchers that isolated Taxol. In 1985 it was discovered that camptothecin inhibited the enzyme DNA topoisomerase, which is extremely important in cell replication and DNA transcription and recombination. There are several camptothecin-derived drugs, including Topotecan from SmithKline Beecham, CPT-11 from Diichi in Japan, GG211 by Glaxo, and 9-nitrocamptothecin (Rubitecan) from Super-Gen (Moss 1998).

### Rubitecan

The drug Rubitecan (previously known as RFS-2000) is another promising drug for treating pancreatic cancer. In a study on a group of 60 evaluative patients with end-stage pancreatic cancer who were treated with this experimental drug, 31.7% responded favorably with a median survival of 18.6 months. Another 31.7% had stabilized disease with a median survival of 9.7 months. Nonresponders (36.6%) lived 6.8 months, with no deaths attributable to treatment (Stehlin et al. 1999). Typically, pancreatic cancer patients live from 3-12 months following diagnosis. It is hoped that combining Rubitecan with other cancer therapies may provide some hope; in addition, pancreatic cancer patients (diagnosed earlier in the disease process) are expected to respond better than those with more advanced disease.

Rubitecan is usually administered orally on an outpatient basis, and can produce side effects described as relatively benign including hematological toxicities, cystitis [bladder irritation], and gastrointestinal complaints.

**Oncophage**

An experimental pancreatic cancer vaccine is being tested by Antigenics. The vaccine is based on technology that uses heat shock proteins (HSPs). HSPs are naturally formed whenever a cell is stressed by factors such as heat, cold, or glucose or oxygen deprivation. Most tumors release a constant flow of necrotic (dead) cells, exposing their HSPs, which are bound to peptides, to the immune system. The HSP-peptide complex stimulates precisely targeted cytotoxic T-cells and nonspecific natural killer (NK) cells. Antigenics makes personalized vaccines from the cells of surgically removed tumors.

**GM-CSF Vaccine**

The GM-CSF vaccine consists of tumor cell lines that are genetically engineered to produce the immune system-stimulating growth factor known as granulocyte-macrophage colony-stimulating factor (GM-CSF). The rationale behind this type of vaccine is that the immune system would recognize the pancreatic cancer cells as foreign and mount an attack against them.

The GM-CSF vaccine was used on 14 patients with pancreatic cancer whose tumors had been surgically removed. The patients received varying amounts of vaccine for 8 weeks after surgery. Twelve of the patients also received 6 months of chemotherapy and radiation therapy. One month following the chemotherapy and radiation, six patients who were in remission received additional vaccinations. Three patients receiving one of the higher vaccine dosages showed an immune response to their tumor cells and experienced a disease-free survival time of at least 25 months following their diagnosis. This vaccine is deemed safe, without side effects, and the response appears to be dose-dependent (Jaffee et al. 2001).

A clinical trial involving 48 patients with pancreatic cancer that were vaccinated by injection of synthetic mutant Ras peptides in combination with granulocyte-macrophage colony-stimulating factor (GM-CSF) were carried out. Peptide-specific immunity was induced in 25 of 43 (58%) patients, indicating that the vaccine used is very potent and capable of eliciting immune responses even in patients with end-stage disease. Patients with advanced cancer demonstrating an immune response to the peptide vaccine showed prolonged survival (an average of 148 days) from the start of treatment compared to nonresponders (average survival of 61 days) (Gjertsen et al. 2001).

**Oryx-015**

Onyx Pharmaceuticals have developed a recombinant adenovirus that destroys malignant tissue while sparing normal cells. The Onyx-015 (CI-1042) Phase I and II pancreatic trials have been closed, and the results are pending. This drug is being tested at the University of California-San Francisco.

**TNP-470**

A study investigated the effects of the angiogenesis inhibitor TNP-470 on human pancreatic cancer cells in vitro and in vivo. Treatment with TNP-470 significantly reduced new angiogenesis in tumors of all three human pancreatic cancer cell lines. TNP-470 reduced tumor growth and metastatic spread of pancreatic cancer in vivo. This was probably due to the antiproliferative effect of the agent on endothelial cells rather than to the direct inhibition of pancreatic cancer cell growth (Hotz et al. 2001).

**R115777**

Pancreatic cancer cells often proliferate via the farnesyl transferase pathway. The Ras protein attaches to the inner cell membrane through a lipid (fat) called farnesyl. The first attachment step is catalyzed by the enzyme farnesyl transferase. After attachment, the Ras protein is phosphorylated by tyrosine kinase, which activates other kinases in a chain of events that stimulates cell growth. Mutant Ras proteins continuously stimulate cell growth causing excessive cell proliferation resulting in tumors.

The experimental drug R115777 functions as a specific farnesyl transferase inhibitor. The clinical trials are conducted by the National Cancer Institute (NCI) (Prevost et al. 1999).

Several therapeutic strategies are being explored for the treatment of pancreatic cancer, including: Statin drugs, such as Lovastatin; COX-2 inhibitors, such as Lodine, Nimesulide and Sulindac; and Metformin, a drug used in Europe for diabetes.

There is evidence in the scientific literature that the proper combination of cell differentiating agents and chemotherapy may slow the progression of pancreatic cancer.

**Statin Drugs**

Statins have been found to have a number of beneficial effects in addition to their ability to lower plasma LDL-cholesterol. They have been found to reduce the markers of inflammation. Statins, and particularly lipophilic statins, in general inhibit cell proliferation, seemingly by multifaceted mechanisms, including:

- Inhibition of cell cycle progression
- Induction of apoptosis (programmed cell death)
- Reduction of cyclooxygenase-2 activity
- Enhancement of angiogenesis (new blood vessel growth)
- Inhibition of G protein prenylation through a reduction of farnesylation and geranylgeranylation by inhibition of the synthesis of a number of small prenylated GTPases (which are derived from cholesterol and mevalonate) involved in cell growth, motility, and invasion (Sumi et al. 1992; 1994)

This effect has been used to demonstrate that statins are anti-carcinogenic in vitro and in animals (Davignon et al. 2001).

**Lovastatin**

Lovastatin was shown to inhibit proliferation of two pancreatic carcinoma cell lines with p21-ras oncogenes (Muller et al. 1998). Lovastatin augmented, by up to fivefold, the cancer cell-killing effect of Sulindac, a drug with COX-2 inhibiting properties. In this study, three different colon cancer cell lines were killed (made to undergo programmed cell death) by depriving them of COX-2. When Lovastatin was added to the COX-2 inhibitor, the kill rate was increased by up to five times (Agarwal et al. 1999).
The effects of two HMG-CoA reductase inhibitors (Fluvastatin and Fovastatin) on invasion of human pancreatic cancer (PANC-1 cells) were examined. The results suggest that HMG-CoA reductase inhibitors affect RhoA translocation and activation by preventing geranylgeranylation, which results in inhibition of epidermal growth factor (EGF)-induced invasiveness of human pancreatic cancer cells (Kusama et al. 2001).

**COX-2 Inhibitors**

Cyclooxygenase is an enzyme that converts arachidonic acid into prostaglandins, thromboxanes, and other eicosanoids. Cyclooxygenase-1 (COX-1) forms prostaglandins that stimulate the synthesis of protective mucus in the stomach and small intestines. Cyclooxygenase-2 (COX-2) is induced by tissue injury and leads to inflammation and pain. Several types of human tumors over-express COX-2, but not COX-1, and experiments demonstrate a central role of COX-2 in experimental tumor development. COX-2 produces prostaglandins that inhibit apoptosis and stimulate angiogenesis. Nonselective NSAIDs inhibit both COX-1 and COX-2 and can cause platelet dysfunction, gastrointestinal ulceration, and kidney damage. Selective COX-2 inhibitors, such as meloxicam, celecoxib (Celebrex), and rofecoxib (Vioxx), are NSAIDs that have been modified chemically to preferentially inhibit COX-2, but not COX-1, and are currently being investigated for use in cancer treatment (Fosslien 2000).
Since 1997, a wealth of clinical research has confirmed that COX-2 is elevated in many cancers, including pancreatic cancer, and that COX-2 inhibitors are useful in treating cancer.
An article in the journal Cancer Research reported that COX-2 levels in pancreatic cancer cells are 60 times greater than in adjacent normal tissue (Tucker et al. 1999).A study in the journal Cancer Research found COX-2 expression in 14 of 21 (67%) pancreatic carcinomas. Two NSAIDs, sulindac sulfide and NS398, produced a dose-dependent inhibition of cell proliferation in all pancreatic cell lines tested (Molina et al. 1999).
Strong expression of COX-2 protein was present in 23 of 52 (44%) pancreatic carcinomas, a moderate expression was present in 24 (46%), and a weak expression was present in five (10%). In contrast, benign tumors showed weak expression or no expression of COX-2, and only islet cells displayed COX-2 expression in normal pancreatic tissues (Okami et al. 1999).
The general COX inhibitor, indomethacin (Indocin and Indomethacin capsules), and the COX-2 specific inhibitor NS-398 were evaluated on four pancreatic cancer cell lines. Both agents inhibited cellular proliferation and growth and induced apoptosis (programmed cell death) (Ding et al. 2000a).
The mechanism of NSAIDs on COX-2 gene expression was investigated. NSAIDs were found to have a complicated effect on phospholipase enzymes, which results in depriving COX-2 of its substrate, arachidonic acid, which is needed to produce inflammatory prostaglandins (Yuan et al. 2000).
A study in the journal Cancer examined 70 surgically resected pancreatic cancers at the National Cancer Center Hospital in Tokyo. Marked COX-2 expression was observed in 57% (24 of 42) of pancreatic duct cell carcinomas, in

58% (11 of 19) of adenomas, and in 70% (7 of 10) of adenocarcinomas of intraductal papillary mucinous tumors. All four pancreatic cancer cell lines expressed COX-2 protein weakly or strongly, and the inhibitory effect of aspirin on cell growth was correlated with the expression of COX-2 (Kokawa et al. 2001).

**Lodine**

Lodine XL (extended release form) is an arthritis drug approved by the FDA that interferes with COX- 2 metabolic processes. The maximum dosage for Lodine is 1000 mg daily. The most convenient dosing schedule for the patient involves prescribing 2 Lodine XL 500-mg tablets in a single daily dose. As with any NSAID, extreme caution and physician supervision are necessary. The most common complaints associated with Lodine XL use relate to the gastrointestinal tract (PDR 2002). Serious gastrointestinal toxicity, such as perforation, ulceration, and bleeding, can occur in patients treated chronically with NSAID therapy. Serious renal and hepatic reactions have been rarely reported. Lodine XL should not be given to patients who have previously shown hypersensitivity to it or in whom aspirin or other NSAIDs induce asthma, rhinitis, urticaria, or other allergic reactions. Fatal asthmatic reactions have been reported in such patients receiving NSAIDs.

**Nimesulide**

Nimesulide is a safer COX-2 inhibitor approved for use in overseas countries, but not currently approved by the FDA. Several studies have shown nimesulide to be useful in controlling the pain associated with cancer (Gallucci et al. 1992; Corli et al. 1993; Toscani et al. 1993). Nimesulide is available from Mexican pharmacies and European pharmacies. The suggested dose for nimesulide is two 100-mg tablets daily.

**Celecoxib**

Celecoxib (Celebrex) is a COX-2 inhibitor that has been approved for use to relieve the signs and symptoms of rheumatoid arthritis and osteoarthritis (PDR 2002). Published articles describe experiments in which celecoxib was shown to be effective in preventing several drug-induced cancers.

Celecoxib given daily in the diet significantly inhibited the induction of rat mammary tumors by 7,12-dimethylbenz (a)anthracene (DMBA), a tumor-inducing drug. Tumors continued to grow actively in control rats fed chow diet only. In contrast, the celecoxib-supplemented diet significantly decreased the size of the mammary tumors over the 6-week treatment period, resulting in an average reduction in tumor volume of approximately 32%. Tumor regression occurred in 90% of the rats. In addition, new tumors continued to emerge in the control group, in contrast to their significantly reduced numbers in the celecoxib-treated group over the same time period (Alshafie et al. 2000).

In an almost identical experiment with celecoxib- and ibuprofen-fed rats with mammary tumors induced by DMBA, dietary administration of celecoxib produced striking reductions in the incidence, multiplicity, and volume of breast tumors relative to the control group (68%, 86%, and 81%, respectively). Ibuprofen also produced significant effects, but of lesser magnitude (40%, 52%, and 57%, respectively) (Harris et al. 2000).

In an article in the journal Carcinogenesis, celecoxib reduced the number and multiplicity of skin cancers induced by UV light by 56% as compared to the controls (Pentland et al. 1999).

Vioxx (rofecoxib) is another NSAID and COX-2 inhibitor approved for the treatment of osteoarthritis inflammation and pain.

**Sulindac**

Sulindac is an anti-inflammatory UNSAID that has been shown to have a protective effect against the incidence of and mortality associated with colorectal cancer. Sulindac (and two other COX inhibitors, indomethacin and NS-398) inhibited cell growth in both COX-2-positive and COX-2-negative pancreatic tumor cell lines (Yip-Schneider et al. 2000). Treatment with both Sulindac and green tea extract significantly reduced the number of tumors in mice with multiple intestinal neoplasia. Green tea and sulindac alone resulted in a reduction in the number of tumors (Suganuma et al. 2001).

A COX-2 inhibitor and a statin drug may be prescribed to pancreatic cancer patients (in addition to other therapies) for a period of 3 months. Two possible dosing schedules that could be used include: 1000 mg daily of Lodine XL, and 80 mg daily of Mevacor (lovastatin) or Lipitor. Blood tests to assess liver and kidney function are critical in protecting against potential side effects. To ascertain efficacy, regular CA-19.9 serum tests and imagery testing are recommended.

COX-2 inhibiting drugs can be prescribed along with a statin drug as an adjuvant therapy.

## Silymarin, Curcumin

Both silymarin (found in the herb milk thistle) and curcumin (found in the spice turmeric) are selective inhibitors of cyclooxygenase (COX) and may be beneficial in preventing and treating pancreatic cancer (Cuendet et al. 2000). We suggest that high-dose curcumin be initiated 2-4 weeks after cytotoxic chemotherapy has been concluded in those with pancreatic cancer.

## Metformin

Metformin is a drug used to treat diabetes that has been used for more than 20 years in Canada and Europe and more recently in Japan. Metformin lowers elevated glucose levels, but does not cause hypoglycemia in nondiabetic patients. Metformin is available from the FDA only for diabetic patients with severe symptoms that are not controlled by diet and who cannot take insulin.

In an article in the journal Pancreas, the effect of islet hormones on pancreatic cancer cells in vitro was investigated. Insulin (but not somatostatin and glucagon) induced pancreatic cancer cell growth. Insulin also significantly enhanced glucose utilization of pancreatic cancer cells before it enhanced cell proliferation. These findings suggest that insulin stimulates proliferation and glucose utilization in pancreatic cancer cells (Ding et al. 2000b).

In a study in the journal Gastroenterology, Metformin was investigated in two groups of high-fat-fed hamsters. One group received Metformin in drinking water for life, and the other group served as a control. All hamsters were treated with a known pancreatic carcinogen. Although 50% of the hamsters in the high-fat group developed malignant lesions, none were found in the Metformin group. Also, significantly more hyperplastic and premalignant lesions, most of which were found within the islets, were detected in the high-fat group (8.6 lesions per hamster) than in the high-fat and Metformin group (1.8 lesions per hamster). It was proposed that this mechanism might explain the association between pancreatic cancer and obesity that is usually associated with peripheral insulin resistance (Schneider et al. 2001).

Several herbs has also been demonstrated to possess anticancer or immune-modulating properties. Certain utritional therapies have also demonstrated varying degrees of efficacy against pancreatic cancer cells. Specific doses of these nutrients for treating pancreatic cancers are also provided. These therapies may all be combined with the subject cardiac glycosides in treating pancreatic cancer.

## Enzymes

In an extraordinary study by Dr. Nicholas Gonzalez, 11 patients with pancreatic cancer were treated with large doses of pancreatic enzymes, nutritional supplements, "detoxification" procedures (including coffee enemas), and an organic diet. Of the 11 patients, nine (81%) survived 1 year, five (45%) survived 2 years, and four survived 3 years. At the time the study was published, two patients were alive and doing well: one at 3 years and the other at 4 years. This pilot study suggested that an aggressive nutritional therapy with large doses of pancreatic enzymes led to significantly increased survival over what would normally be expected for patients with inoperable pancreatic cancer (Gonzalez et al. 1999).

Dr. John Beard, who published The Enzyme Theory of Cancer in 1911, first proposed the concept of using pancreatic digestive enzymes to treat cancer. However, enzyme therapy was largely forgotten after his death in 1923, except by a few alternative therapists. While in medical school, Dr. Gonzalez met Dr. William Donald Kelley, a Texas dentist who had been treating cancer patients with enzymes for more than 20 years. After reviewing his medical records, Dr. Gonzalez found many cases that had followed Dr. Kelley's program and lived far beyond what would be expected with this disease. In comparison, a trial of 126 patients with pancreatic cancer treated with the newly approved drug, gemcitabine, reported that not a single patient lived longer than 19 months.

As a result of the pilot study, the National Cancer Institute (NCI) and the National Center for Complementary and Alternative Medicine approved funding for a large-scale clinical trial comparing Dr. Gonzalez's nutritional therapy against gemcitabine in the treatment of inoperable pancreatic cancer. This study has full FDA approval and is being conducted under the Department of Oncology and the Department of Surgical Oncology at Columbia Presbyterian Medical Center in New York.

## Monoterpenes

Monoterpenes are non-nutritive dietary components found in the essential oils of citrus fruits and other plants. A number of dietary monoterpenes have antitumor activity. Several mechanisms of action may account for the antitumor activities of monoterpenes, including:

- Induction of hepatic Phase II carcinogen-metabolizing enzymes, resulting in carcinogen detoxification

- Induction of apoptosis (programmed cell death)
- Inhibition of cell growth by inhibiting the prenylation of Ras and other proteins
- Suppression of hepatic HMG-CoA reductase activity, a rate-limiting step in cholesterol synthesis

Monoterpenes appear to act through multiple mechanisms in the prevention and chemotherapy of cancer. Although the mechanism of action has yet to be elucidated, the monoterpenes, limonene, and perillyl alcohol have a profound antitumor activity on pancreatic cancer (Elson et al. 1994; Gelb et al. 1995; Crowell et al. 1996; Gould 1997; Bardon et al. 1998; Crowell 1999).

**Limonene**

The growth inhibitory effects of limonene and other monoterpenes (including perillyl alcohol) on pancreatic carcinoma cells carrying a K-Ras mutation were examined. Limonene caused an approximately 50% growth reduction. Although effective in inhibiting the growth of tumor cells harboring activated ras oncogenes, limonene and perillyl alcohol are unlikely to act by inhibiting Ras function (Karlson et al. 1996).

**Perillyl Alcohol**

Perillyl alcohol is a monoterpene consisting of two isoprene units manufactured in the melavonate pathway. It is found in small concentrations in the essential oils of lavender, peppermint, spearmint, sage, cherries, cranberries, perilla, lemongrass, wild bergamot, gingergrass, savin, and caraway and celery seeds (Belanger 1998).

Perillyl alcohol was shown to reduce the growth of pancreatic tumors injected into hamsters to less than half that of controls. Moreover, 16% of pancreatic tumors treated with perillyl alcohol completely regressed, whereas no control tumors regressed (Stark et al. 1995).

Perillyl alcohol and perillic acid are metabolites of limonene. Limonene is only a weak inhibitor of the isoprenylation enzymes of Ras and other proteins, whereas perillyl alcohol and perillic acid are more potent inhibitors (Hardcastle et al. 1999).

One study of perillyl alcohol found that Ras prenylation by farnesyl protein transferase (FPTase) was inhibited by 17% and RhoA prenylation by geranylgeranyl protein transferase (GGPTase) was inhibited by 28%. FPTase and GGPTase are the two enzymes involved in the process of attaching Ras proteins to the inner membrane of the cell. By inhibiting this first step, the mutated Ras proteins are not able to continuously stimulate cell growth causing excessive cell proliferation resulting in tumors (Broitman et al. 1996).

Further investigation into the effect of perillyl alcohol on prenylation enzymes, however, found that perillyl alcohol inhibited farnesylation and MAP kinase phosphorylation in H-Ras, but not in K-Ras (Stayrook et al. 1998). Perillyl alcohol induces apoptosis without affecting the rate of DNA synthesis in both liver and pancreatic tumor cells (Crowell et al. 1996).

In an article in the journal Carcinogenesis, Staybrook et al. (1997) concluded that the inhibitory effects of perillyl alcohol on pancreatic cell growth were due to a stimulation of apoptosis by increasing the proapoptotic protein, Bak.

In the first Phase I trial of perillyl alcohol, 18 patients with advanced malignancies were treated with perillyl alcohol 3 times daily. One patient with ovarian cancer experienced a decline in CA-125 and several others experienced a stabilization of their disease for up to 6 months. Due to the short half-life of the metabolites, a more frequent dosing schedule is recommended (Ripple et al. 1998).

In the second Phase I trial, perillyl alcohol was administered 4 times a day. Sixteen patients with advanced refractory malignancies were treated. Evidence of antitumor activity was seen in a patient with metastatic colorectal cancer who has an ongoing near-complete response of greater than 2-year duration. Several other patients were studied for greater than or equal to 6 months with stable disease (Ripple et al. 2000).

The predominant toxicity of perillyl alcohol seen during both trials was gastrointestinal (nausea, vomiting, satiety, and eructation), limiting the dose.

**Borage Oil**

Gamma linolenic acid (GLA) is a fatty acid that has been shown to inhibit the growth and metastasis of a variety of tumor cells, including pancreatic cancer. Gamma linolenic acid has also been shown to inhibit angiogenesis, the formation of new blood vessels, which is an essential feature of malignant tumor development (Cai et al. 1999).

GLA treatment has been shown to dramatically change tissue perfusion, especially in liver and pancreatic tumors, even at low doses, and these changes may predict response to GLA therapy (Kairemo et al. 1997).

The lithium salt of gamma-linolenic acid (Li-GLA) was tested in mice implanted with pancreatic cancer cells. Administration of Li-GLA into the tumor was associated with a significant antitumor effect (Ravichandran et al. 1998a;

1998b).

Gamma-linolenic acid (GLA) has been found to kill about 40 different human cancer cell lines in vitro without harming normal cells. The lithium salt of GLA (LiGLA) was administered intravenously to 48 patients with inoperable pancreatic cancer in two different treatment centers. Analysis of the results showed that the highest doses of LiGLA were associated with longer survival times as compared with the lowest doses (Fearon et al. 1996).

Cyclooxygenase-2 (COX-2) and lipooxygenase inhibitors are being used to interfere with the growth of several different cell lines including pancreatic cancer. One experimental approach is to use the 5-lipooxygenase inhibitor, MK886, along with borage oil. Other approaches to suppressing COX-2 could be the use of one of the new COX-2 inhibiting drugs used to treat rheumatoid arthritis; or fish oil supplements providing at least 2400 mg of EPA and 1800 mg DHA daily; or importing the drug nimesulide from Europe or Mexico for personal use (Anderson et al. 1998).

**Fish Oil**

Patients with advanced cancer usually experience weight loss and wasting (cachexia) and often fail to gain weight with conventional nutritional support. Several studies have shown that supplementation with fish oils containing the essential fatty acids EPA (eicosapentaenoic acid) and DHA (docosahexaenoic acid) have been helpful and may even reverse the cachexia.

The biological activity of both lipid mobilizing factor and protein mobilizing factor was shown to be attenuated by eicosapentaenoic acid (EPA). Clinical studies show that EPA is able to stabilize the rate of weight loss, as well as adipose tissue and muscle mass, in cachectic patients with pancreatic cancer (Tisdale 1999).

In a study by Barber et al. (1999), 20 patients with pancreatic cancer were asked to consume 2 cans of a fish oil-enriched nutritional supplement daily in addition to their normal food intake. Each can contained 16.1 grams of protein and 1.09 grams of EPA. At the beginning of the study, all patients were losing weight at baseline at a median rate of 2.9 kg a month. After administration of the fish oil-enriched supplement, patients had a significant weight gain at both 3 and 7 weeks (Barber et al. 1999).

In another study, after 3 weeks of an EPA-enriched supplement, the body weight of the cancer patients had increased and the energy expenditure in response to feeding had risen significantly, such that it was no different from baseline healthy control values (Barber et al. 2000).

Wigmore et al. (1996) reported a study of 18 patients with pancreatic cancer who received dietary supplementation orally with fish oil capsules (1 gram each) containing eicosapentaenoic acid (EPA) 18% and docosahexaenoic acid (DHA) 12%. Patients had a median weight loss of 2.9 kg a month prior to supplementation. At a median of 3 months after commencement of fish oil supplementation, patients had a median weight gain of 0.3 kg a month (Wigmore et al. 1996).

Eicosapentaenoic acid (EPA) has also been shown to have an inhibitory effect on the growth of several pancreatic cancer cell lines in vitro. A time- and dose-dependent decrease in cell count and viability in cultures of pancreatic cancer cells supplemented with EPA was found to occur (Lai et al. 1996).

A number of polyunsaturated fatty acids have been shown to inhibit the growth of malignant cells in vitro. Lauric, stearic, palmitic, oleic, linoleic, alpha-linolenic, gamma-linolenic, arachidonic, docosahexaenoic, and eicosapentaenoic acids all had an inhibitory effect on the growth of human pancreatic cancer cells, with EPA being the most potent. Monounsaturated or saturated fatty acids were not inhibitory. The action of EPA could be reversed with the antioxidant vitamin E acetate or with oleic acid (Falconer et al. 1994).

**Soy.**

Genistein has potent tumor growth-regulating characteristics. The effect of genistein has been attributed partially to its tyrosine kinase-regulating properties, resulting in cell-cycle arrest and limited angiogenesis. In a study of nonoxidative ribose synthesis in pancreatic cancer cells, genistein was shown to control tumor growth primarily through the regulation of glucose metabolism (Boros et al. 2001).

Dietary protease inhibitors, such as the soybean-derived Bowman-Birk inhibitor and chymotrypsin inhibitor 1 from potatoes, can be powerful anticarcinogenic agents. Human populations known to have high concentrations of protease inhibitors in the diet have low overall cancer mortality rates (Anon. 1989).

If the pathology report shows the pancreatic cancer cells to have a mutated p53 oncogene, or if there is no p53 detected, then high-dose genistein therapy may be appropriate. The suggested dose is 5 capsules, 4 times a day, of the 700-mg Ultra-Soy Extract supplement that provides over 2800 mg daily of genistein. If the pathology report shows a functional p53, then genistein is far less effective in arresting cell growth.

Refer to the protocol titled Cancer Treatment: The Critical Factors for information about the special pathology report (immunohistochemistry) that determines tumor cell p53 status.

## Vitamin A

A Phase II pilot study of 23 patients with pancreatic cancer was conducted to evaluate beta-interferon and retinol palmitate (vitamin A) with chemotherapy: eight patients responded (35%), and eight patients had stable disease (35%). Median time to progression and survival for all patients were, respectively, 6.1 months and 11 months. Toxicity was high, but patients who had responses and disease stabilization had prolonged symptom palliation (Recchia et al. 1998).

A new retinoid, mofarotene (RO40-8757), was compared with other retinoids on nine pancreatic cancer cell lines. After treatment with each retinoid, antiproliferative effect was determined. Mofarotene was found to inhibit the growth of pancreatic cancer cells by inducing G1-phase cell cycle-inhibitory factors (p21, p27, and hypophosphorylated form of Rb protein) and is considered to be a useful agent for pancreatic cancer treatment (Kawa et al. 1997a).

## Vitamin D

In tumor-bearing mice given a vitamin D analogue (EB 1089) 3 times weekly for 4-6 weeks, tumor growth was significantly inhibited in the absence of hypercalcemia (Colston et al. 1997b). Vitamin D was also shown to inhibit cell growth in pancreatic cancer lines by up-regulating cyclin-dependent kinase inhibitors (p21 and p27) (Kawa et al. 1997).

Zugmaier et al. (1996) reported that vitamin D analogues together with retinoids were shown to inhibit the growth of human pancreatic cancer cells. A study by Kawa et al. (1996) also reported that a new vitamin D3 analogue, 22-oxa-1,25-dihydroxyvitamin D3 (22-oxa-calcitriol), was tested and found to markedly inhibit the proliferation (three of nine cell lines) and cause a G1 phase cell cycle arrest in pancreatic cancer cells.

## Green Tea

A review article on green tea stated that "pancreatic cancer studies hint at an inverse association in two of three studies" (Bushman 1998). Black and green tea extracts and components of these extracts were examined in vitro for their effect on tumor cell growth. Results showed inhibition (approximately 90%) of cell growth in pancreatic tumor cells by black and green tea extracts (0.02%). Black and green tea extracts also decreased the expression of the K-ras gene (Lyn-Cook et al. 1999).

An article in the journal Pancreas described two experiments in which green tea extract was tested in hamsters with pancreatic cancer. In the first experiment, pancreatic cancer was induced by a drug. Fewer of the green tea extract-treated hamsters had pancreatic cancers (54% versus 33%) and the average number of tumors was less (1 versus 0.5 per hamster). In the second experiment, pancreatic cancers were transplanted onto the back of hamsters. Tumor growth was similar in both groups until 11 weeks after transplantation, when inhibition of tumor growth became apparent in the green tea extract group. At 13 weeks, the average tumor volume in the green tea extract group was significantly smaller than that in the control group. These results demonstrated that green tea extract has an inhibitory effect on the process of pancreatic carcinogenesis and on tumor promotion of transplanted pancreatic cancer (Hiura et al. 1997).

## Quercetin

Quercetin, a bioflavonoid found in many vegetables, has been studied for use in many types of cancer, including breast, bladder, and colon cancer. Its use in pancreatic cancer has yet to be examined, but many of the cancer pathogenesis mechanisms are similar (Lamson et al. 2000). Quercetin was also found to down-regulate the expression of mutant p53 protein in human breast cancer lines to nearly undetectable levels (Avila et al. 1994). In addition, quercetin has been found to arrest the expression of p21-ras oncogenes in colon cancer cell lines (Ranelletti et al. 2000).

A study reported in the Japanese journal Cancer Research found that quercetin was a potent inhibitor of cyclooxygenase-2 (COX-2) transcription in human colon cancer cells (Mutoh et al. 2000).

## Selenium

A study in the journal Carcinogenesis tested the effects of beta-carotene and selenium on mice with pancreatic tumors induced by azaserine. Beta-carotene and selenium were found to have inhibitory effects on pancreatic cancer growth (Appel et al. 1996). Also, a diet high in selenium was found to significantly reduce the number of drug-induced pancreatic cancers in female Syrian golden hamsters (Kise et al. 1990).

## Mistletoe

In a Phase I/II study, the effect of mistletoe (Eurixor) treatment was evaluated in 16 patients with pancreatic

cancer. Mistletoe was administered twice a week by subcutaneous injection. Apart from one anaphylactic reaction, which necessitated suspension of treatment for a few days, no severe side effects were observed. Eight patients (50%) showed a CT-verified status of "no change" (according to the World Health Organization criteria) for at least 8 weeks. Median survival time in all patients was 5.6 months (range =1.5-26.5 months). All except two patients claimed that mistletoe had a positive effect on their quality of life, with an obvious decline only during the last weeks of life. These results indicate that mistletoe can stabilize quality of life and therefore may help patients to maintain adequate life quality in their few remaining months (Friess et al. 1996).

Another study described a patient with inoperable cancer of the pancreas who developed marked eosinophilia during treatment (on day 22) with injections of Viscum album (mistletoe). Furthermore, histology performed on day 28 revealed accumulation of eosinophils in the pancreas. Although the overall clinical course of the disease was rapidly progressive, temporary stabilization of the patient's general condition during mistletoe treatment was observed (Huber et al. 2000).

The following section provides some detailed dosage information for several pancreatic cancer treatment protocols, all of which may be combined with the subject cardiac glycosides in treating pancreatic cancers.

**Suppressing ras Oncogene Expression**

Ras oncogenes play a central role in the regulation of cancer cell cycle and proliferation. Mutations in genes that encode Ras proteins have been intimately associated with unregulated cell proliferation (i.e., cancer). The vast majority of pancreatic cancers over-express the ras oncogene. There is a class of cholesterol-lowering drugs known as the statins that have been shown to inhibit the activity of ras oncogenes. One or more of the following statin drugs may be used to inhibit the activity of ras oncogenes:

- Lovastatin, 40 mg twice daily
- Zocor, 40 mg twice daily
- Pravachol, 40 mg once a day

These statin drugs may produce toxic effects in a minority of patients. Physician oversight and monthly blood tests to evaluate liver function are suggested.

In addition to statin drug therapy, to further suppress ras oncogene expression, patients may supplement therapy with Aged Garlic Extract (e.g. about 1200 mg a day). One1000-mg caplets per day of Kyolic-brand aged garlic may be used.

**Inhibiting the COX-2 Enzyme**

Pancreatic cancer cells use the COX-2 enzyme as biological fuel to hyper-proliferate. Levels of the COX-2 enzyme may be 60 times higher in pancreatic cancer cells compared to adjacent healthy tissue. Suppressing the COX-2 enzyme can dramatically inhibit pancreatic cancer cell propagation. One of the following COX-2 inhibiting drugs may be used:

- Lodine XL, 1000 mg once daily
- Celebrex, 100-200 mg every 12 hours
- Vioxx, 12.5-25 mg once daily

**Blocking Cancer Cell Growth Signals**

Pancreatic cancer cells are highly resistant to chemotherapy. The reason for this is that pancreatic cancer cells possess multiple survival mechanisms that enable them to readily mutate in order to escape cell regulatory control. The following supplements might help block growth signals used by cancer cells to escape eradication by chemotherapy and other cytotoxic cancer therapies. These supplements have also displayed anti-angiogenesis properties. Some of these supplements may be best initiated 1 week after cessation of chemotherapy if one believes that the antioxidant component of these nutrients will protect cancer cells from the effects of chemotherapy drug(s):

Soy Extract (40% isoflavones), five 700-mg capsules taken 4 times a day. The only soy extract providing this high potency of soy isoflavones is a product called Ultra Soy. Note that isoflavones from soy have antioxidant properties.

Curcumin, 900 mg with 5 mg of bioperine (an alkaloid from Piper nigrum), 3 capsules, 2-4 times a day, taken 2 hours apart from medications. (Super Curcumin with Bioperine is a formulated product that contains this recommended dosage). Curcumin is a potent antioxidant.

Green tea extract, five 350-mg capsules with each meal (3 meals a day). Each capsule should be standardized

to provide a minimum of 100 mg of epigallocatechin gallate (EGCG). It is the EGCG fraction of green tea that has shown the most active anticancer effects. These are available in decaffeinated form for those who are sensitive to caffeine or who want to take the less stimulating decaffeinated green tea extract capsules in their evening dose. (Green tea is also a potent antioxidant).

Silibinin, two 250-mg capsules 3 times a day.

**Maintaining Optimal Fatty Acid Balance**

Several studies show that gamma linolenic acid (GLA) inhibits pancreatic cancer cell growth. Fish oil concentrate high in EPA and DHA has been shown to reverse weight loss (cachexia), reduce levels of growth-promoting prostaglandin E2, and inhibit ras oncogene expression. Thus in one embodiment, patients are administered an encapsulated borage oil supplement that provides a minimum of 1500 mg of gamma-linolenic acid (GLA) each day. In another embodiment, patients are administered a fish oil concentrate that provides 3200 mg of EPA and 2400 mg of DHA each day.

**Inducing Cancer Cell Differentiation and Apoptosis**

Cancer cells fail to properly differentiate and undergo normal apoptotic processes (programmed cell death). Vitamin A and vitamin D drug analogs are suggested. Accutane (13-cis-retinoic acid) is an example of a vitamin A drug that could benefit many pancreatic cancer patients. In one embodiment, supplement with 100,000-300,000 IU of emulsified vitamin A liquid drops may be used by a patient. If a vitamin D analog drug is not available, supplement with 6000 IU of vitamin D3, although monthly blood tests may be necessary to guard against hypercalcemia and kidney damage.

**Pancreatic Enzyme Therapy**

A pilot study published in June 1999 indicated that aggressive nutritional therapy dramatically prolonged survival of pancreatic cancer patients. This approach is currently being evaluated in a large-scale study, funded by the National Institutes of Health's National Center for Complementary and Alternative Medicine with collaboration from the National Cancer Institute. A key component of this program is the ingestion of large quantities of pork pancreas enzymes throughout the day.

Saruc et al. (Pancreas. 28(4): 401-12, May 2004) recently reported that pancreatic enzyme extract improves survival in murine pancreatic cancer. Briefly, the malignant human PC cell line AsPC1 was transplanted into the pancreas of male beige XID nude mice that were treated or not with porcine pancreatic enzyme extract (PPE) in drinking water. The survival, size, and volume of tumors, plasma pancreatic enzyme levels, fecal fat, and urine were examined as were the expression of transforming growth factor alpha, insulinlike growth factor-I, epidermal growth factor, epidermal growth factor receptor, apoptosis, and proliferation rate of tumor cells. The results show that: PPE-treated mice survived significantly longer than the control group ($P < 0.002$). Tumors in the PPE-treated group were significantly smaller than in the control group. All mice in the control group showed steatorrhea, hyperglucosuria, hyperbilirubinuria, and ketonuria at early stages of tumor growth, whereas only a few in the treated group showed some of these abnormalities at the final stage. There were no differences in the expression of growth factors, epidermal growth factor receptor, or the apoptotic rate between the tumors of treated and control mice. Thus, the treatment with PPE significantly prolongs the survival of mice with human PC xenografts and slows the tumor growth. The data indicate that the beneficial effect of PPE on survival is primarily related to the nutritional advantage of the treated mice. The preparation of PPE was provided in the study.

To implement, a patient may take a minimum of five 425-mg pork pancreas enzyme capsules 6 times a day. Take pancreatic enzymes with meals and in-between meals around the clock. Additional doses of enzymes may be administered at night. After the first several months, the dose of pancreatic enzymes is usually reduced significantly. In some embodiments, patients take the equivalent of over 100 pork pancreas enzyme capsules a day.

Other pharmaceutical agents that may be used in the subject combination therapy with cardiac glycosides include, merely to illustrate: aminoglutethimide, amsacrine, anastrozole, asparaginase, bcg, bicalutamide, bleomycin, buserelin, busulfan, campothecin, capecitabine, carboplatin, carmustine, chlorambucil, cisplatin, cladribine, clodronate, colchicine, cyclophosphamide, cyproterone, cytarabine, dacarbazine, dactinomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, ironotecan, letrozole, leucovorin, leuprolide, levamisole, lomustine, mechlorethamine, medroxyprogesterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.

These anti-cancer agents may be categorized by their mechanism of action into, for example, following groups: anti-metabolites/anti-cancer agents, such as pyrimidine analogs (5-fluorouracil, floxuridine, capecitabine, gemcitabine and cytarabine) and purine analogs, folate antagonists and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristin, vinblastin, nocodazole, epothilones and navelbine, epidipodophyllotoxins (teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, docetaxel, doxorubicin, epirubicin, hexamethylmelamineoxaliplatin, iphosphamide, melphalan, merchlorethamine, mitomycin, mitoxantrone, nitrosourea, paclitaxel, plicamycin, procarbazine, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes - dacarbazinine (DTIC); antiproliferative/antimitotic antimetabolites such as folic acid analogs (methotrexate); platinum coordination complexes (cisplatin, carboplatin), procarbazine; hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, COX-2 inhibitors, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); anti-angiogenic compounds (TNP-470, genistein) and growth factor inhibitors (vascular endothelial growth factor (VEGF) inhibitors, fibroblast growth factor (FGF) inhibitors, epidermal growth factor (EGF) inhibitors); angiotensin receptor blocker; nitric oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin, irinotecan (CPT-11) and mitoxantrone, topotecan, irinotecan), corticosteroids (cortisone, dexamethasone, hydrocortisone, methylpednisolone, prednisone, and prenisolone); growth factor signal transduction kinase inhibitors; mitochondrial dysfunction inducers and caspase activators; chromatin disruptors.

Many combinatorial therapies have been developed in prior art, including but not limited to those listed in Table 1.

Table 1: Exemplary conventional combination cancer chemotherapy

| Name | Therapeutic agents |
|---|---|
| ABV | Doxorubicin, Bleomycin, Vinblastine |
| ABVD | Doxorubicin, Bleomycin, Vinblastine, Dacarbazine |
| AC (Breast) | Doxorubicin, Cyclophosphamide |
| AC (Sarcoma) | Doxorubicin, Cisplatin |
| AC (Neuroblastoma) | Cyclophosphamide, Doxorubicin |
| ACE | Cyclophosphamide, Doxorubicin, Etoposide |
| ACe | Cyclophosphamide, Doxorubicin |
| AD | Doxorubicin, Dacarbazine |
| AP | Doxorubicin, Cisplatin |
| ARAC-DNR | Cytarabine, Daunorubicin |
| B-CAVe | Bleomycin, Lomustine, Doxorubicin, Vinblastine |
| BCVPP | Carmustine, Cyclophosphamide, Vinblastine, Procarbazine, Prednisone |
| BEACOPP | Bleomycin, Etoposide, Doxorubicin, Cyclophosphamide, Vincristine, Procarbazine, Prednisone, Filgrastim |
| BEP | Bleomycin, Etoposide, Cisplatin |
| BIP | Bleomycin, Cisplatin, Ifosfamide, Mesna |

(continued)

| Name | Therapeutic agents |
|---|---|
| BOMP | Bleomycin, Vincristine, Cisplatin, Mitomycin |
| CA | Cytarabine, Asparaginase |
| CABO | Cisplatin, Methotrexate, Bleomycin, Vincristine |
| CAF | Cyclophosphamide, Doxorubicin, Fluorouracil |
| CAL-G | Cyclophosphamide, Daunorubicin, Vincristine, Prednisone, Asparaginase |
| CAMP | Cyclophosphamide, Doxorubicin, Methotrexate, Procarbazine |
| CAP | Cyclophosphamide, Doxorubicin, Cisplatin |
| CaT | Carboplatin, Paclitaxel |
| CAV | Cyclophosphamide, Doxorubicin, Vincristine |
| CAVE ADD | CAV and Etoposide |
| CA-VP16 | Cyclophosphamide, Doxorubicin, Etoposide |
| CC | Cyclophosphamide, Carboplatin |
| CDDP/VP-16 | Cisplatin, Etoposide |
| CEF | Cyclophosphamide, Epirubicin, Fluorouracil |
| CEPP(B) | Cyclophosphamide, Etoposide, Prednisone, with or without/ Bleomycin |
| CEV | Cyclophosphamide, Etoposide, Vincristine |
| CF | Cisplatin, Fluorouracil or Carboplatin Fluorouracil |
| CHAP | Cyclophosphamide or Cyclophosphamide, Altretamine, Doxorubicin, Cisplatin |
| ChlVPP | Chlorambucil, Vinblastine, Procarbazine, Prednisone |
| CHOP | Cyclophosphamide, Doxorubicin, Vincristine, Prednisone |
| CHOP-BLEO | Add Bleomycin to CHOP |
| CISCA | Cyclophosphamide, Doxorubicin, Cisplatin |
| CLD-BOMP | Bleomycin, Cisplatin, Vincristine, Mitomycin |
| CMF | Methotrexate, Fluorouracil, Cyclophosphamide |
| CMFP | Cyclophosphamide, Methotrexate, Fluorouracil, Prednisone |
| CMFVP | Cyclophosphamide, Methotrexate, Fluorouracil, Vincristine, Prednisone |
| CMV | Cisplatin, Methotrexate, Vinblastine |
| CNF | Cyclophosphamide, Mitoxantrone, Fluorouracil |
| CNOP | Cyclophosphamide, Mitoxantrone, Vincristine, Prednisone |
| COB | Cisplatin, Vincristine, Bleomycin |
| CODE | Cisplatin, Vincristine, Doxorubicin, Etoposide |
| COMLA | Cyclophosphamide, Vincristine, Methotrexate, Leucovorin, Cytarabine |
| COMP | Cyclophosphamide, Vincristine, Methotrexate, Prednisone |
| Cooper Regimen | Cyclophosphamide, Methotrexate, Fluorouracil, Vincristine, Prednisone |
| COP | Cyclophosphamide, Vincristine, Prednisone |
| COPE | Cyclophosphamide, Vincristine, Cisplatin, Etoposide |
| COPP | Cyclophosphamide, Vincristine, Procarbazine, Prednisone |
| CP(Chronic lymphocytic leukemia) | Chlorambucil, Prednisone |

(continued)

| Name | Therapeutic agents |
|---|---|
| CP (Ovarian Cancer) | Cyclophosphamide, Cisplatin |
| CT | Cisplatin, Paclitaxel |
| CVD | Cisplatin, Vinblastine, Dacarbazine |
| CVI | Carboplatin, Etoposide, Ifosfamide, Mesna |
| CVP | Cyclophosphamide, Vincristine, Prednisome |
| CVPP | Lomustine, Procarbazine, Prednisone |
| CYVADIC | Cyclophosphamide, Vincristine, Doxorubicin, Dacarbazine |
| DA | Daunorubicin, Cytarabine |
| DAT | Daunorubicin, Cytarabine, Thioguanine |
| DAV | Daunorubicin, Cytarabine, Etoposide |
| DCT | Daunorubicin, Cytarabine, Thioguanine |
| DHAP | Cisplatin, Cytarabine, Dexamethasone |
| DI | Doxorubicin, Ifosfamide |
| DTIC/Tamoxifen | Dacarbazine, Tamoxifen |
| DVP | Daunorubicin, Vincristine, Prednisone |
| EAP | Etoposide, Doxorubicin, Cisplatin |
| EC | Etoposide, Carboplatin |
| EFP | Etoposie, Fluorouracil, Cisplatin |
| ELF | Etoposide, Leucovorin, Fluorouracil |
| EMA 86 | Mitoxantrone, Etoposide, Cytarabine |
| EP | Etoposide, Cisplatin |
| EVA | Etoposide, Vinblastine |
| FAC | Fluorouracil, Doxorubicin, Cyclophosphamide |
| FAM | Fluorouracil, Doxorubicin, Mitomycin |
| FAMTX | Methotrexate, Leucovorin, Doxorubicin |
| FAP | Fluorouracil, Doxorubicin, Cisplatin |
| F-CL | Fluorouracil, Leucovorin |
| FEC | Fluorouracil, Cyclophosphamide, Epirubicin |
| FED | Fluorouracil, Etoposide, Cisplatin |
| FL | Flutamide, Leuprolide |
| FZ | Flutamide, Goserelin acetate implant |
| HDMTX | Methotrexate, Leucovorin |
| Hexa-CAF | Altretamine, Cyclophosphamide, Methotrexate, Fluorouracil |
| ICE-T | Ifosfamide, Carboplatin, Etoposide, Paclitaxel, Mesna |
| IDMTX/6-MP | Methotrexate, Mercaptopurine, Leucovorin |
| IE | Ifosfamide, Etoposie, Mesna |
| IfoVP | Ifosfamide, Etoposide, Mesna |
| IPA | Ifosfamide, Cisplatin, Doxorubicin |

(continued)

| Name | Therapeutic agents |
|---|---|
| M-2 | Vincristine, Carmustine, Cyclophosphamide, Prednisone, Melphalan |
| MAC-III | Methotrexate, Leucovorin, Dactinomycin, Cyclophosphamide |
| MACC | Methotrexate, Doxorubicin, Cyclophosphamide, Lomustine |
| MACOP-B | Methotrexate, Leucovorin, Doxorubicin, Cyclophosphamide, Vincristine, Bleomycin, Prednisone |
| MAID | Mesna, Doxorubicin, Ifosfamide, Dacarbazine |
| m-BACOD | Bleomycin, Doxorubicin, Cyclophosphamide, Vincristine, Dexamethasone, Methotrexate, Leucovorin |
| MBC | Methotrexate, Bleomycin, Cisplatin |
| MC | Mitoxantrone, Cytarabine |
| MF | Methotrexate, Fluorouracil, Leucovorin |
| MICE | Ifosfamide, Carboplatin, Etoposide, Mesna |
| MINE | Mesna, Ifosfamide, Mitoxantrone, Etoposide |
| mini-BEAM | Carmustine, Etoposide, Cytarabine, Melphalan |
| MOBP | Bleomycin, Vincristine, Cisplatin, Mitomycin |
| MOP | Mechlorethamine, Vincristine, Procarbazine |
| MOPP | Mechlorethamine, Vincristine, Procarbazine, Prednisone |
| MOPP/ABV | Mechlorethamine, Vincristine, Procarbazine, Prednisone, Doxorubicin, Bleomycin, Vinblastine |
| MP (multiple myeloma) | Melphalan, Prednisone |
| MP (prostate cancer) | Mitoxantrone, Prednisone |
| MTX/6-MO | Methotrexate, Mercaptopurine |
| MTX/6-MP/VP | Methotrexate, Mercaptopurine, Vincristine, Prednisone |
| MTX-CDDPAdr | Methotrexate, Leucovorin, Cisplatin, Doxorubicin |
| MV (breast cancer) | Mitomycin, Vinblastine |
| MV (acute myelocytic leukemia) | Mitoxantrone, Etoposide |
| M-VAC Methotrexate | Vinblastine, Doxorubicin, Cisplatin |
| MVP Mitomycin | Vinblastine, Cisplatin |
| MVPP | Mechlorethamine, Vinblastine, Procarbazine, Prednisone |
| NFL | Mitoxantrone, Fluorouracil, Leucovorin |
| NOVP | Mitoxantrone, Vinblastine, Vincristine |
| OPA | Vincristine, Prednisone, Doxorubicin |
| OPPA | Add Procarbazine to OPA. |
| PAC | Cisplatin, Doxorubicin |
| PAC-I | Cisplatin, Doxorubicin, Cyclophosphamide |
| PA-CI | Cisplatin, Doxorubicin |
| PC | Paclitaxel, Carboplatin or Paclitaxel, Cisplatin |
| PCV | Lomustine, Procarbazine, Vincristine |
| PE | Paclitaxel, Estramustine |

(continued)

| Name | Therapeutic agents |
|---|---|
| PFL | Cisplatin, Fluorouracil, Leucovorin |
| POC | Prednisone, Vincristine, Lomustine |
| ProMACE | Prednisone, Methotrexate, Leucovorin, Doxorubicin, Cyclophosphamide, Etoposide |
| ProMACE/cytaBOM | Prednisone, Doxorubicin, Cyclophosphamide, Etoposide, Cytarabine, Bleomycin, Vincristine, Methotrexate, Leucovorin, Cotrimoxazole |
| PRoMACE/MOPP | Prednisone, Doxorubicin, Cyclophosphamide, Etoposide, Mechlorethamine, Vincristine, Procarbazine, Methotrexate, Leucovorin |
| Pt/VM | Cisplatin, Teniposide |
| PVA | Prednisone, Vincristine, Asparaginase |
| PVB | Cisplatin, Vinblastine, Bleomycin |
| PVDA | Prednisone, Vincristine, Daunorubicin, Asparaginase |
| SMF | Streptozocin, Mitomycin, Fluorouracil |
| TAD | Mechlorethamine, Doxorubicin, Vinblastine, |
| TCF | Vincristine, Bleomycin, Etoposide, Prednisone Paclitaxel, Cisplatin, Fluorouracil |
| TIP | Paclitaxel, Ifosfamide, Mesna, Cisplatin |
| TTT | Methotrexate, Cytarabine, Hydrocortisone |
| Topo/CTX | Cyclophosphamide, Topotecan, Mesna |
| VAB-6 | Cyclophosphamide, Dactinomycin, Vinblastine, Cisplatin, Bleomycin |
| VAC | Vincristine, Dactinomycin, Cyclophosphamide |
| VACAdr | Vincristine, Cyclophosphamide, Doxorubicin, Dactinomycin, Vincristine |
| VAD | Vincristine, Doxorubicin, Dexamethasone |
| VATH | Vinblastine, Doxorubicin, Thiotepa, Flouxymesterone |
| VBAP | Vincristine, Carmustine, Doxorubicin, Prednisone |
| VBCMP | Vincristine, Carmustine, Melphalan, Cyclophosphamide, Prednisone |
| VC | Vinorelbine, Cisplatin |
| VCAP | Vincristine, Cyclophosphamide, Doxorubicin, Prednisone |
| VD | Vinorelbine, Doxorubicin |
| VelP | Vinblastine, Cisplatin, Ifosfamide, Mesna |
| VIP | Etoposide, Cisplatin, Ifosfamide, Mesna |
| VM | Mitomycin, Vinblastine |
| VMCP | Vincristine, Melphalan, Cyclophosphamide, Prednisone |
| VP | Etoposide, Cisplatin |
| V-TAD | Etoposide, Thioguanine, Daunorubicin, Cytarabine |
| 5 + 2 | Cytarabine, Daunorubicin, Mitoxantrone |
| 7 + 3 | Cytarabine with/, Daunorubicin or Idarubicin or Mitoxantrone |
| "8 in 1" | Methylprednisolone, Vincristine, Lomustine, Procarbazine, Hydroxyurea, Cisplatin, Cytarabine, Dacarbazine |

In addition to conventional anti-cancer agents, the agent of the subject method can also be compounds and antisense RNA, RNAi or other polynucleotides to inhibit the expression of the cellular components that contribute to unwanted cellular proliferation that are targets of conventional chemotherapy. Such targets are, merely to illustrate, growth factors, growth factor receptors, cell cycle regulatory proteins, transcription factors, or signal transduction kinases.

The method of present invention is advantageous over combination therapies know in the art because it allows conventional anti-cancer agent to exert greater effect at lower dosage. In preferred embodiment of the present invention, the effective dose ($ED_{50}$) for a anti-cancer agent or combination of conventional anti-cancer agents when used in combination with a cardiac glycoside is at least 2-fold, preferably 5-fold less than the $ED_{50}$ for the anti-cancer agent alone. Conversely, the therapeutic index (TI) for such anti-cancer agent or combination of such anti-cancer agent when used in combination with a cardiac glycoside is at least 2-fold, preferably 5-fold greater than the TI for conventional anti-cancer agent regimen alone.

C. Other combinations

Also disclosed herein is the combination of a cardiac glycoside with radiation therapies, including ionizing radiation, gamma radiation, or particle beams.

D. Administration

The cardiac glycoside, or a combination containing a cardiac glycoside may be administered orally, parenterally by intravenous injection, transdermally, by pulmonary inhalation, by intravaginal or intrarectal insertion, by subcutaneous implantation, intramuscular injection or by injection directly into an affected tissue, as for example by injection into a tumor site. In some instances the materials may be applied topically at the time surgery is carried out. In another instance the topical administration may be ophthalmic, with direct application of the therapeutic composition to the eye.

In a preferred embodiment, the subject cardiac glycoside compounds are administered to a patient by using osmotic pumps, such as Alzet® Model 2002 osmotic pump. Osmotic pumps provides continuous delivery of test agents, thereby eliminating the need for frequent, round-the-clock injections. With sizes small enough even for use in mice or young rats, these implantable pumps have proven invaluable in predictably sustaining compounds at therapeutic levels, avoiding potentially toxic or misleading side effects.

To meet different therapeutic needs, ALZET's osmotic pumps are available in a variety of sizes, pumping rates, and durations. At present, at least ten different pump models are available in three sizes (corresponding to reservoir volumes of 100 μL, 200 μL and 2 mL) with delivery rates between 0.25 μL/hr and 10 μL/hr and durations between one day to four weeks.

While the pumping rate of each commercial model is fixed at manufacture, the dose of agent delivered can be adjusted by varying the concentration of agent with which each pump is filled. Provided that the animal is of sufficient size, multiple pumps may be implanted simultaneously to achieve higher delivery rates than are attainable with a single pump. For more prolonged delivery, pumps may be serially implanted with no ill effects. Alternatively, larger pumps for larger patients, including human and other non-human mammals may be custom manufactured by scaling up the smaller models.

The materials are formulated to suit the desired route of administration. The formulation may comprise suitable excipients include pharmaceutically acceptable buffers, stabilizers, local anesthetics, and the like that are well known in the art. For parenteral administration, an exemplary formulation may be a sterile solution or suspension; For oral dosage, a syrup, tablet or palatable solution; for topical application, a lotion, cream, spray or ointment; for administration by inhalation, a microcrystalline powder or a solution suitable for nebulization; for intravaginal or intrarectal administration, pessaries, suppositories, creams or foams. Preferably, the route of administration is parenteral, more preferably intravenous.

EXAMPLES

The following examples are for illustrative purpose only, and should in no way be construed to be limiting in any respect of the claimed invention.

The ememplary cardiac glycosides used in following studies are referred to as BNC1 and BNC4.

BNC1 is ouabain or g-Strophanthin (STRODIVAL®), which is not a bufadienolide according to the invention, and has been used for treating myocardial infarction. It is a colorless crystal with predicted $IC_{50}$ of about 0.009-0.35 μg/mL and max. plasma concentration of about 0.03 μg/mL. According to the literature, its plasma half-life in human is about 20 hours, with a range of between 5-50 hours. Its common formulation is injectable. The typical dose for current indication (i.v.) is about 0.25 mg, up to 0.5 mg /day.

BNC4 is proscillaridin (TALUSIN®), which has been approved for treating chronic cardiac insufficiency in Europe.

It is a colorless crystal with predicted $IC_{50}$ of about 0.002-0.008 μg/mL and max. plasma concentration of about 0.001 μg/mL. According to the literature, its plasma half-life in human is about 40 hours. Its common available formulation is a tablet of 0.25 or 0.5 mg. The typical dose for current indication (p.o.) is about 1.5 mg /day.

Some of the examples described below took advantage of the Sentinel Line™ of reporter cell lines for assaying / monitoring gene activity in response to drug treatment. Some details of the Sentinel Lines™ construction are described below.

**Example I. Sentinel Line Plasmid Construction and Virus Preparation**

Figure 1 is a schematic drawing of the Sentinel Line promoter trap system, and its use in identifying regulated genetic sites and in reporting pathway activity. Briefly, the promoter-less selection markers (either positive or negative selection markers, or both) and reporter genes (such as beta-gal) are put in a retroviral vector (or other suitable vectors), which can be used to infect target cells. The randomly inserted retroviral vectors may be so positioned that an active upstream heterologous promoter may initiate the transcription and translation of the selectable markers and reporter gene(s). The expression of such selectable markers and/or reporter genes is indicative of active genetic sites in the particular host cell.

In one exemplary embodiment, the promoter trap vector BV7 was derived from retrovirus vector pQCXIX (BD Biosciences Clontech) by replacing sequence in between packaging signal ($Psi^+$) and 3' LTR with a cassette in an opposite orientation, which contains a splice acceptor sequence derived from mouse engrailed 2 gene (SA/en2), an internal ribosomal entry site (IRES), a LacZ gene, a second IRES, and fusion gene TK:Sh encoding herpes virus thymidine kinase (HSV-tk) and phleomycin followed by a SV40 polyadenylation site. BV7 was constructed by a three-way ligation of three equal molar DNA fragments. Fragment 1 was a 5 kb vector backbone derived from pQCXIX by cutting plasmid DNA extracted from a Dam- bacterial strain with Xho I and Cla I (Dam- bacterial strain was needed here because Cla I is blocked by overlapping Dam methylation). Fragment 2 was a 2.5 kb fragment containing an IRES and a TK:Sh fusion gene derived from plasmid pIREStksh by cutting Dam- plasmid DNA with Cla I and Mlu I. pIREStksh was constructed by cloning TK:Sh fragment from pMODtksh (InvivoGen) into pIRES (BD Biosciences Clontech). Fragment 3 was a 5.8 kb SA/en2-IRES-LacZ fragment derived from plasmid pBSen2IRESLacZ by cutting with BssH II (compatible end to Mlu I) and Xho I. pBSen2IRESLacZ was constructed by cloning IRES fragment from pIRES and LacZ fragment from pMODLacZ (InvivoGen) into plasmid pBSen2.

To prepare virus, packaging cell line 293T was co-transfected with three plasmids BV7, pVSV-G (BD Biosciences Clontech) and pGag-Pol (BD Biosciences Clontech) in equal molar concentrations by using Lipofectamine 2000 (InvitroGen) according to manufacturer's protocol. First virus "soup" (supernatant) was collected 48 hours after transfection, second virus "soup" was collected 24 hours later. Virus particles were pelleted by centrifuging at 25,000 rpm for 2 hours at 4°C. Virus pellets were re-dissolved into DMEM/10% FBS by shaking overnight. Concentrated virus solution was aliquot and used freshly or frozen at -80 °C.

**Example II. Sentinel Line Generation**

Target cells were plated in 150 mm tissue culture dishes at a density of about $1 \times 10^6$/ plate. The following morning cells were infected with 250 μl of Bionaut Virus #7 (BV7) as prepared in Example I, and after 48 hr incubation, 20 μg/ml of phleomycin was added. 4 days later, media was changed to a reduced serum (2%FBS) DMEM to allow the cells to rest. 48h later, ganciclovir (GCV) (0.4μM, sigma) was added for 4 days (media was refreshed on day 2). One more round of phleomycin selection followed (20 μg/ml phleomycin for 3 days). Upon completion, media was changed to 20%FBS DMEM to facilitate the outgrowths of the clones. 10 days later, clones were picked and expanded for further analysis and screening.

Usig this method, several Sentinel Lines were generated to report activity of genetic sites activated by hypoxia pathways (Figure 3). These Sentinel lines were generated by transfecting A549 (NSCLC lung cancer) and Panc-1 (pancreatic cancer) cell lines with the subject gene-trap vectors containing *E. coli* LacZ-encoded β-galactosidase (β-gal) as the reporter gene (Figure 3). The β-gal activity in Sentinel Lines (green) was measured by flow cytometry using a fluorogenic substrate fluoresescein di-beta-D-galactopyranoside (FDG). The autofluorescence of untransfected control cells is shown in purple. The graphs indicate frequency of cells (y-axis) and intensity of fluorescence (x-axis) in log scale. The bar charts on the right depict median fluorescent units of the FACS curves. They indicate a high level of reporter activity at the targeted site.

**Example III. Cell Culture**

All cell lines can be purchased from ATCC, or obtained from other sources.

A549 (CCL-185) and Panc-1 (CRL-1469) were cultured in Dulbecco's Modified Eagle's Medium (DMEM). Media

was supplemented with 10% FBS (Hyclone; SH30070.03), 100 μg/ml penicillin and 50 μg/ml streptomycin (Hyclone).

In some experiments, cells were subject to hypoxia in culture. To induce hypoxia conditions, cells were placed in a Billups-Rothenberg modular incubator chamber and flushed with artificial atmosphere gas mixture (5% $CO_2$, 1% $O_2$, and balance $N_2$). The hypoxia chamber was then placed in a 37°C incubator. L-mimosine (Sigma, M-0253) was used to induce hypoxia-like HIF1-alpha expression. Proteasome inhibitor, MG132 (Calbiochem, 474791), was used to protect the degradation of HIF1-alpha. Cycloheximide (Sigma, 4859) was used to inhibit new protein synthesis of H1F1-alpha. Catalase (Sigma, C3515) was used to inhibit reactive oxygen species (ROS) production.

**Example IV. Identification of Trapped Genes**

Once a Sentinel Line with a desired characteristics was established, it might be helpful to determine the active promoter under which control the markers / reporter genes are expressed. To do so, total RNAs were extracted from cultured Sentinel Line cells by using, for example, RNA-Bee RNA Isolation Reagent (TEL-TEST, Inc.) according to the manufacturer's instructions. Five prime ends of the genes that were disrupted by the trap vector BV7 were amplified by using BD SMART RACE cDNA Amplification Kit (BD Biosciences Clontech) according to the manufacturer's protocol. Briefly, 1 μg total RNA prepared above was reverse-transcribed and extended by using BD PowerScriptase with 5' CDS primer and BD SMART II Oligo both provided by the kit. PCR amplification were carried out by using BD Advantage 2 Polymerase Mix with Universal Primer A Mix provided by the kit and BV7 specific primer 5'Rsa/ires (gacgcggatcttccgggtaccgagctcc, 28 mer). 5'Rsa/ires located in the junction of SA/en2 and IRES with the first 7 nucleotides matching the last 7 nucleotides of SA/en2 in complementary strand. 5' RACE products were cloned into the TA cloning vector pCR2.1 (InvitroGen) and sequenced. The sequences of the RACE products were analyzed by using the BLAST program to search for homologous sequences in the database of GenBank. Only those hits which contained the transcript part of SA/en2 were considered as trapped genes.

Using this method, the upstream promoters of several Sentinel Lines generated in Example II were identified (see below). The identity of these trapped genes validate the clinical relevance of these Sentinel Lines™, and can be used as biomarkers and surrogate endpoints in clinical trials.

| Sentinel >Lines | Genetic Sites | Gene Profile |
|---|---|---|
| A7N1C1 | Essential Antioxidant | Tumor cell-specific gene, over expressed in lung tumor cells |
| A7N1C6 | Chr. 3, BAC, map to 3p | novel |
| A7I1C1 | Pyruvate Kinase (PKM 2), Chr. 15 | Described biomarker for NSCLC |
| A6E2A4 | 6q14.2-16.1 | Potent angiogenic activity |
| A7I1D1 | Chr. 7, BAC | novel |

**Example V. Sentinel Line Reporter Assays**

The expression level of beta-galactosidase gene in sentinel lines was determined by using a fluorescent substrate fluorescein di-B-D-Galactopyranside (FDG, Marker Gene Tech, #M0250) introduced into cells by hypotonic shock. Cleavage by beta-galactosidase results in the production of free fluorescein, which is unable to cross the plasma membrane and is trapped inside the beta-gal positive cells. Briefly, the cells to be analyzed are trypsinized, and resuspended in PBS containing 2 mM FDG (diluted from a 10mM stock prepared in 8:1:1 mixture of water: ethanol: DMSO): The cells were then shocked for 4 minutes at 37°C and transferred to FACS tubes containing cold 1 × PBS on ice. Samples were kept on ice for 30 minutes and analyzed by FACS in FL1 channel.

**Example VI. Testing Standard Chemotherapeutic Agents**

Sentinel Line cells with beta-galactosidase reporter gene were plated at 1 x $10^5$ cells / 10 cm dish. After overnight incubation, the cells were treated with standard chemotherapeutic agents, such as mitoxantrone (8 nM), paclitaxel (1.5 nM), carboplatin (15 pM), gemcitabine (2.5 nM), in combination with one or more BNC compounds, such as BNC1 (10 nM), BNC2 (2 μM), BNC3 (100 μM) and BNC4 (10 nM), or a targeted drug, Iressa (4 μM). After 40 hrs, the cells were trypsinized and the expression level of reporter gene was determined by FDG loading.

When tested in the Sentinel Lines, mitoxanthrone, paclitaxel, and carboplatin each showed increases in cell death and reporter activity (see Figure 6). No effect had been expected from the cytotoxic agents because of their nonspecific mechanisms of action (MOA), making their increased reporter activity in HIF-sensitive cell lines surprising. These results provide a previously unexplored link between the development of chemotherapy resistance and induction

of the hypoxia response in cells treated with anti-neoplastic agents. Iressa, on the other hand, a known blocker of EGFR-mediated HIF-1 induction, showed a reduction in reporter activity when tested. The Sentinel Lines thus provide a means to differentiate between a cytotoxic agent and a targeted drug.

**Example VII. Pharmacokinetic (PK) Analysis**

The following protocol can be used to conduct pharmacokinetic analysis of any compounds of the invention. To illustrate, BNC1 is used as an example.

Nude mice were dosed *i.p.* with 1, 2, or 4 mg/kg of BNC1. Venous blood samples were collected by cardiac puncture at the following 8 time points: 5 min, 15 min, 30 min, 45 min, 1 hr, 2 hr, 4 hr, 8 hr, and 24 hr. For continuous BNC1 treatment, osmotic pumps (such as Alzet® Model 2002) were implanted s.c. between the shoulder blades of each mouse. Blood was collected at 24 hr, 48 hr and 72 hr. Triplicate samples per dose (i.e. three mice per time point per dose) were collected for this experiment.

Approximately 0.100 mL of plasma was collected from each mouse using lithium heparin as anticoagulant. The blood samples were processed for plasma as individual samples (no pooling). The samples were frozen at -70°C (±10°C) and transferred on dry ice for analysis by HPLC.

For PK analysis plasma concentrations for each compound at each dose were analyzed by non-compartmental analysis using the software program WinNonlin®. The area under the concentration vs time curve AUC (0-Tf) from time zero to the time of the final quantifiable sample (Tf) was calculated using the linear trapezoid method. AUC is the area under the plasma drug concentration-time curve and is used for the calculation of other PK parameters. The AUC was extrapolated to infinity (0-Inf) by dividing the last measured concentration by the terminal rate constant (k), which was calculated as the slope of the log-linear terminal portion of the plasma concentrations curve using linear regression. The terminal phase half-life ($t_{1/2}$) was calculated as 0.693/k and systemic clearance (Cl) was calculated as the dose(mg/kg) /AUC(Inf). The volume of distribution at steady-state (Vss) was calculated from the formula:

$$V_{ss} = dose(AUMC)/(AUC)^2$$

where *AUMC* is the area under the first moment curve (concentration multiplied by time versus time plot) and *AUC* is the area under the concentration versus time curve. The observed maximum plasma concentration ($C_{max}$) was obtained by inspection of the concentration curve, and $T_{max}$ is the time at when the maximum concentration occurred.

Figure 8 shows the result of a representative pharmacokinetic analysis of BNC1 delivered by osmotic pumps. Osmotic pumps (Model 2002, Alzet Inc) containing 200 µl of BNC1 at 50, 30 or 20 mg/ml in 50% DMSO were implanted subcutaneously into nude mice. Mice were sacrificed after 24, 48 or 168 hrs, and plasma was extracted and analyzed for BNC1 by LC-MS. The values shown are average of 3 animals per point.

**Example VIII. Human Tumor Xenograft Models**

Female nude mice (nu/nu) between 5 and 6 weeks of age weighing approximately 20 g were implanted subcutaneously (s.c.) by trocar with fragments of human tumors harvested from s.c. grown tumors in nude mice hosts. When the tumors were approximately 60-75 mg in size (about 10-15 days following inoculation), the animals were pair-matched into treatment and control groups. Each group contains 8-10 mice, each of which was ear tagged and followed throughout the experiment.

The administration of drugs or controls began the day the animals were pair-matched (Day 1). Pumps (Alzet® Model 2002) with a flow rate of 0.5 µl/hr were implanted s.c. between the shoulder blades of each mice. Mice were weighed and tumor measurements were obtained using calipers twice weekly, starting Day 1. These tumor measurements were converted to mg tumor weight by standard formula, ($W^2$ x L)/2. The experiment is terminated when the control group tumor size reached an average of about 1 gram. Upon termination, the mice were weighed, sacrificed and their tumors excised. The tumors were weighed and the mean tumor weight per group was calculated. The change in mean treated tumor weight/the change in mean control tumor weight x 100 (dT/dC) is subtracted from 100% to give the tumor growth inhibition (TGI) for each group.

**Example IX. Cardiac Glycoside Compounds Inhibits HIF-1α Expression**

As part of an attempt to study the mechanism of the inhibitory function on pancreatic cancers by the subject cardiac glycosides, the inventors found that cardiac glycoside compounds of the invention targets and inhibits the

expression of HIF 1α based on Western Blot analysis using antibodies specific for HIT1α.

In one study, reporter tumor cell line A549(ROS) were incubated in normoxia in the absence (control) or presence of different amounts of BNC1 for 4 hrs. Thirty minutes prior to the termination of incubation period, 2,7-dichlorofluorescin diacetate (CFH-DA, 10 mM) was added to the cells and incubated for the last 30 min at 37°C. The ROS levels were determined by FACS analysis. HIF1α protein accumulation in pancreatic cancer cell line Panc-1 cells was determined by western blotting after incubating the cells for 4 hrs in normoxia (21% $O_2$) or hypoxia (1% $O_2$) in the presence or absence of BNC1. Figure 4 indicates that BNC1 induces ROS production (at least as evidenced by the A549(ROS) Sentinel Lines), and inhibits HIF1α protein accumulation in the test cells.

Figure 5 also demonstrates that the cardiac glycoside compounds BNC1 and BNC4 directly or indirectly inhibits in tumor cells the secretion of the angiogenesis factor VEGF, which is a downstream effector of HIF-1α. In contrast, other non-cardiac glycoside compounds, BNC2, BNC3 and BNC5, do not inhibit, and in fact greatly enhances VEGF secretion.

Figure 15 compared the ability of BNC1 and BNC4 in inhibiting hypoxia-mediated HIF1α induction in certain human tumor cells, including the pancreatic cancer cell line Panc-1. The figures show result of immunoblotting for HIF-1α, HIF-1β and β-actin (control) expression in Caki-1 or Panc-1 cells treated with BNC1 or BNC4 under hypoxia. The results indicate that BNC4 is even more potent (about 10-times more potent) than BNC1 in inhibiting HIF-1α expression.

**Example X. Neutralization of Gemcitabine-induced Stress Response as Measured in A549 Sentinel Line**

The cardiac glycoside compounds of the invention were found to be able to neutralize Gemcitabine-induced stress response in tumor cells, as measured in A549 Sentinal Lines.

In experiments of Figure 7, the A549 sentinel line was incubated with Gemcitabine in the presence or absence of indicated Bionaut compounds (including the cardiac glycoside compound BNC4) for 40 hrs. The reporter activity was measured by FACS analysis.

It is evident that at least BNC4 can significantly shift the reporter activity to the left, such that Gemcitabine and BNC4-treated cells had the same reporter activity as that of the control cells. In contrast, cells treated with only Gemcitabine showed elevated reporter activity.

**Example XI. Effect of BNC1 Alone or in Combination with Standard Chemotherapy on Growth of Xenografted Human Pancreatic** Tumors in Nude Mice

To test the ability of BNC1 to inhibit xenographic tumor growth in nude mice, either along or in combination with a standard chemotherapeutic agent, such as Gemcitabine, Panc-1 tumors were injected subcutaneously (sc) into the flanks of male nude mice. After the tumors reached 80 mg in size, osmotic pumps (model 2002, Alzet Inc., flow rate 0.5 μl/hr) containing 20 mg/ml of BNC1 were implanted sc on the opposite sides of the mice. The control animals received pumps containing vehicle (50% DMSO in DMEM). The mice treated with standard chemotherapy agent received intraperitoneal injections of Gemcitabine at 40 mg/kg every 3 days for 4 treatments (q3d × 4). Each data point represent average tumor weight (n = 8) and error bars indicate SEM.

Figure 9 indicates that, at the dosage tested, BNC1 alone can significantly reduce tumor growth in this model. This anti-tumor activity is additive when BNC1 is co-administered with a standard chemotherapeutic agent Gemcitabine. Results of the experiment is listed below:

| Group (Animal No.) | Dose / Route | Final Tumor weight Day 25 (Mean) | SEM | %TGI |
|---|---|---|---|---|
| Control (8) | Vehicle /i.v. | 1120.2 | 161.7 | - |
| BNC1(8) | 20 mg/ml; s.c.; C.I. | 200 | 17.9 | 82.15 |
| Gemcitabine (8) | 40 mg/kg; q3dx4 | 701.3 | 72.9 | 37.40 |
| BNC1 + Gem (8) | Combine both | 140.8 | 21.1 | 87.43 |

Similarly, in the experiment of Figure 10, BNC1 (20 mg/ml) was delivered by sc osmotic pumps (model 2002, Alzet Inc.) at 0.5 μl/hr throughout the study. Cytoxan (q1d×1) was injected at 100 mg/kg (Cyt 100) or 300 mg/kg (Cyt 300). The results again shows that BNC1 is a potent anti-tumor agent when used alone, and its effect is additive with other chemotherapeutic agents such as Cytoxan. The result of this study is listed in the table below:

| Group (Animal No.) | Dose / Route | Final Tumor weight Day 22 (Mean) | SEM | %TGI |
|---|---|---|---|---|
| Control (10) | Vehicle / i.v. | 1697.6 | 255.8 | - |
| BNC1 (10) | 20 mg/ml; s.c.; C.I. | 314.9 | 67.6 | 81.45 |
| Cytoxan300 (10) | 300 mg/ml; ip; qd×1 | 93.7 | 24.2 | 94.48 |
| Cytoxan100 (10) | 100 mg/ml; ip; qdx2 | 769 | 103.2 | 54.70 |
| BNC1 + Cytoxan100 (10) | Combine both | 167 | 39.2 | 90.16 |

In yet another experiment, the anti-tumor activity of BNC1 alone or in combination with Carboplatin was tested in A549 human non-small-cell-lung carcinoma. In this experiment, BNC1 (20 mg/ml) was delivered by sc osmotic pumps (model 2002, Alzet Inc.) at 0.5 μl/hr throughout the study. Carboplatin (q1d×1) was injected at 100 mg/kg (Carb).

Figure 11 confirms that either BNC1 alone or in combination with Carboplatin has potent anti-tumor activity in this tumor model. The detailed results of the experiment is listed in the table below:

| Group (Animal No.) | Dose / Route | % Weight Change | Final Tumor weight Day | SEM | %TGI |
|---|---|---|---|---|---|
| Control (8) | Vehicle / i.v. | 21% | 842.6 | 278.1 | - |
| BNC1 (8) | 20 mg/ml; s.c.; C.I. | 21% | 0.0 | 0.0 | 100.00 |
| Carboplatin (8) | 100 mg/kg; ip; qd×1 | 16% | 509.75 | 90.3 | 39.50 |
| BNC1 + Carb (8) | Combine both | 0% | 0.0 | 0.0 | 100.00 |

Since Carboplatin can be used for treatment of pancreatic cancers, the same result is expected if the same therapeutic regimen is applied to pancreatic cancer treatment. Notably, in both the BNC1 and BNC1 / Carb treatment group, none of the experimental animals showed any signs of tumor at the end of the experiment, while all 8 experimental animals in control and Carb only treatment groups developed tumors of significant sizes.

Thus the cardiac glycoside compounds of the invention (e.g. BNC1) either alone or in combination with many commonly used chemotherapeutic agents (e.g. Carboplatin, Gem, Cytoxan, etc.) has potent anti-tumor activities in xenographic animal models of pancreatic cancer and many other cancers.

**Example XII. Determining Minimum Effective Dose**

Given the additive effect of the subject cardiac glycosides with the traditional chemotherapeutic agents, it is desirable to explore the minimal effective doses of the subject cardiac glycosides for use in conjoint therapy with the other anti-tumor agents.

Figure 12 shows the titration of the exemplary cardiac glycoside BNC1 to determine its minimum effective dose, effective against Panc-1 human pancreatic xenograft in nude mice. BNC1 (sc, osmotic pumps) was first tested at 10, 5 and 2 mg/ml. Gem was also included in the experiment as a comparison.

Figure 13 shows that combination therapy using both Gem and BNC1 produces a combination effect, such that sub-optimal doses of both Gem and BNC1, when used together, produce the maximal effect only achieved by higher doses of individual agents alone.

A similar experiment was conducted using BNC1 and 5-FU (another pancreatic cancer drug), and the same combination effect was seen (see Figure 14).

Similar results are also obtained for other compounds (e.g. BNC2) that are not cardiac glycoside compounds (data not shown).

**Example XIII. BNC4 Inhibits HBF-1α Induced under Normoxia by PHD Inhibitor**

As an attempt to study the mechanism of BNC4 inhibition of HM-1α, we tested the ability of BNC1 and BNC4 to inhibit EIF-1α expression induced by a PHD inhibitor, L-mimosone, under normoxia condition.

In the experiment represented in Figure 16, Hep3B cells were grown under normoxia, but were also treated as indicated with 200 μM L-mimosone for 18h in the presence or absence of BNC1 or BNC4. Abundance of HIF1α and β-actin was determined by western blotting:

The results indicate that L-mimosone induced HIF-1α accumulation under normoxia condition, and addition of BNC4 eliminated HIP-1α accumulation by L-mimosone. At the low concentration tested, BNC1 did not appear to have

an effect on HIF-1α accumulation in this experiment. While not wishing to be bound by any particular theory, the fact that BNC4 can inhibit HIF-1α induced under normoxia by PHD inhibitor indicates that the site of action by BNC4 probably lies down stream of prolyl-hydroxylanon.

EQUIVALENTS:

While specific embodiments of the subject inventions are explicitly disclosed herein, the above specification is illustrative and not restrictive. Many variations of the inventions will become apparent to those skilled in the art upon review of this specification and the claims below. The full scope of the inventions should be determined by reference to the claims, along with their full scope of equivalents, and the specification, along with such variations.

## Claims

1. A pharmaceutical formulation comprising a $Na^+/K^+$-ATPase inhibitors in the form of a cardiac glycoside or aglycone form thereof, in combination with an anti-cancer agent, formulated in a pharmaceutically acceptable excipient for use in the treatment of pancreatic cancer, characterise in that said cardiac glycoside or aglycone form thereof comprises a steroid core with a pyrone substituent at C17 (the "bufadienolides form").

2. The pharmaceutical formulation for use according to claim 1, wherein the cardiac glycoside or aglycone form thereof is a cardiac glycoside represented by the general formula:

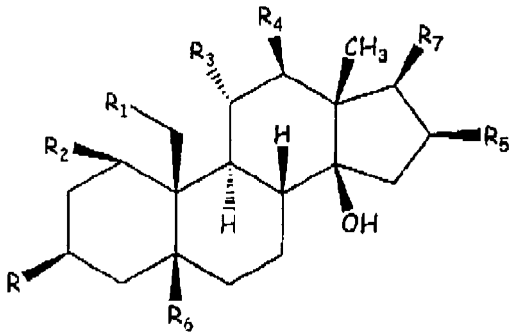

wherein
R represents a glycoside of 1 to 6 sugar residues;
$R_1$ represents hydrogen, -OH or =O;
$R_2$, $R_3$, $R_4$, $R_5$, and $R_6$ each independently represents hydrogen or-OH; and $R_7$ represents

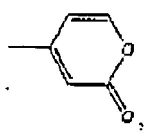

which cardiac glycosides has an IC50 for killing one or more different cancer cell lines of 500 nM or less.

3. The pharmaceutical formulation for use according to claim 1, wherein the cardiac glycoside or aglycone form thereof is proscillaridin.

4. The pharmaceutical formulation for use according to claim 1, wherein said anti-cancer agent is an inhibitor of chromatin function, a DNA topoisomerase inhibitor, a microtubule inhibiting drug, a DNA damaging agent, an antimetabolite, a DNA synthesis inhibitor, or a DNA binding agent.

**5.** The pharmaceutical formulation for use according to claim 1, wherein said anti-cancer agent is gemcitabine, mitoxanthrone, paclitaxel, or carboplatin; and said cardiac glycoside or aglycone form thereof is proscillaridin.

**6.** A kit for use in treating a patient having pancreatic cancer, comprising a $Na^+/K^+$-ATPase inhibitor in the form of a cardiac glycoside or aglycone form thereof, in combination with an anti-cancer agent, each formulated in premeasured doses for administration to the patient, **characterized in that** said cardiac glycoside or aglycone form thereof comprises a steroid core with a pyrone substituent at C17 (the "bufadienolides form").

**7.** The kit for use according to claim 6, wherein the cardiac glycoside or aglycone form thereof is a cardiac glycoside represented by the general formula:

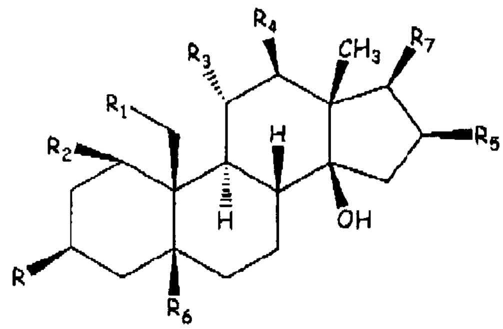

wherein
R represents a glycoside of 1 to 6 sugar residues;
$R^1$ represents hydrogen, -OH or =O;
$R_2$, $R_3$, $R_4$, $R_5$, and $R_6$ each independently represents hydrogen or-OH; and $R_7$ represents

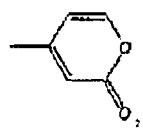

which cardiac glycoside has an IC50 for killing one or more different cancer cell lines of 500 nM or less.

**8.** The kit for use according to claim 6, wherein the cardiac glycoside or aglycone form thereof is proscillaridin.

**9.** The kit for use according to claim 6, wherein said anti-cancer agent is an inhibitor of chromatin function, a DNA topoisomerase inhibitor, a microtubule inhibiting drug, a DNA damaging agent, an antimetabolite, a DNA synthesis inhibitor or a DNA binding agent.

**10.** The kit for use according to claim 6, wherein said anti-cancer agent is gemcitabine, mitoxanthrone, paclitaxel, or carboplatin; and said cardiac glycoside or aglycone form thereof is proscillaridin.

**11.** A $Na^+/K^+$-ATPase inhibitor in the form of a cardiac glycoside or aglycone form thereof for use in combination with an anti-cancer agent for use in the treatment of pancreatic cancer, **characterized in that** said cardiac glycoside or aglycone form thereof comprises a steroid core with a pyrone substituent at C17 (the "bufadienolides form"),

**12.** The cardiac glycoside or aglycone form thereof for use according to claim 11, wherein the cardiac glycoside or aglycone form thereof is a cardiac glycoside represented by the general formula:

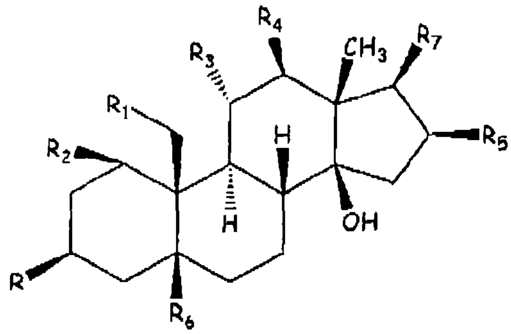

wherein
R represents a glycoside of 1 to 6 sugar residues;
$R_1$ represents hydrogen, -OH or =O;
$R_2$, $R_3$, $R_4$, $R_5$, and $R_6$ each independently represents hydrogen or—OH; and $R_7$ represents

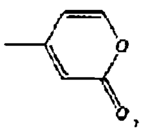

which cardiac glycoside has an IC50 for killing one or more different cancer cell lines of 500 nM: or less.

13. The cardiac glycoside or aglycone form thereof for use according to claim 11, wherein the cardiac glycoside or aglycone form thereof is proscillaridin.

14. The cardiac glycoside or aglycone form thereof for use according to claim 11, wherein said anti-cancer agent is an inhibitor of chromatin function, a DNA topoisomerase inhibitor, a microtubule inhibiting drug, a DNA damaging agent, an antimetabolite, a DNA synthesis inhibitor or a DNA binding agent.

15. The cardiac glycoside or aglycone form thereof for use according to claim 11, wherein said anti-cancer agent is gemcitabine, mitoxanthrone, paclitaxel, or carboplatin; and said cardiac glycoside or aglycone form thereof is proscillaridin.

**Patentansprüche**

1. Pharmazeutische Formulierung, die einen $Na^+/K^+$-ATPase-Inhibitor in Form eines Herzglykosids oder der Aglykon-Form davon in Kombination mit einem Antikrebsmittel umfasst, formuliert in einem pharmazeutisch akzeptablen Exzipienten, zur Verwendung bei der Behandlung von Bauchspeicheldrüsenkrebs, **dadurch gekennzeichnet, dass** das genannte Herzglykosid oder die Aglykon-Form davon einen Steroidkern mit einem Pyron-Substituenten bei C17 umfasst (die "Bufadienoliden-Form").

2. Pharmazeutische Formulierung zur Verwendung nach Anspruch 1, wobei das Herzglykosid oder die Aglykon-Form davon ein Herzglykosid ist, das durch die folgende allgemeine Formel repräsentiert ist:

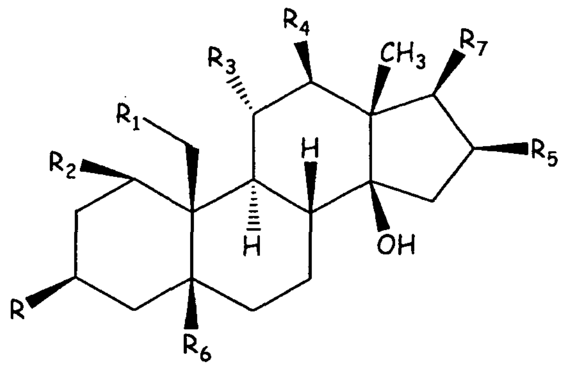

wobei
R ein Glykosid mit 1 bis 6 Zuckerresten repräsentiert;
$R_1$ Wasserstoff, -OH oder =O repräsentiert;
$R_2$, $R_3$, $R_4$, $R_5$ und $R_6$ jeweils unabhängig Wasserstoff oder -OH

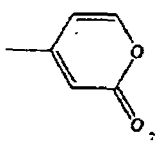

repräsentieren; und $R_7$ Folgendes repräsentiert wobei das Herzglykosid einen IC50 zur Abtötung von einer oder mehren verschiedenen Krebszelllinien von 500 nM oder weniger hat.

**3.** Pharmazeutische Formulierung zur Verwendung nach Anspruch 1, wobei das Herzglykosid oder die Aglykon-Form davon Proscillaridin ist.

**4.** Pharmazeutische Formulierung zur Verwendung nach Anspruch 1, wobei das genannte Antikrebsmittel ein Inhibitor der Chromatinfunktion, ein DNA-Topoisomerase-Inhibitor, ein Mikrotubulus-hemmendes Arzneimittel, ein DNA-schädigendes Mittel, ein Antimetabolit, ein DNA-Synthese-Inhibitor oder ein DNA-Bindemittel ist.

**5.** Pharmazeutische Formulierung zur Verwendung nach Anspruch 1, wobei das genannte Antikrebsmittel Gemcitabin, Mitoxanthron, Paclitaxel oder Carboplatin ist; und das genannte Herzglykosid oder die Aglykon-Form davon Proscillaridin ist.

**6.** Kit zur Verwendung bei der Behandlung eines Patienten mit Bauchspeicheldrüsenkrebs, der einen $Na^+/K^+$-ATPase-Inhibitor in Form eines Herzglykosids oder der Aglykon-Form davon in Kombination mit einem Antikrebsmittel umfasst, die jeweils in zuvor abgemessenen Dosen zur Verabreichung an den Patienten formuliert sind, **dadurch gekennzeichnet, dass** das genannte Herzglykosid oder die Aglykon-Form davon einen Steroidkern mit einem Pyron-Substituenten bei C17 umfasst (die "Bufadienoliden-Form ").

**7.** Kit zur Verwendung nach Anspruch 6, wobei das Herzglykosid oder die Aglykon-Form davon ein Herzglykosid ist, das durch die folgende allgemeine Formel repräsentiert ist:

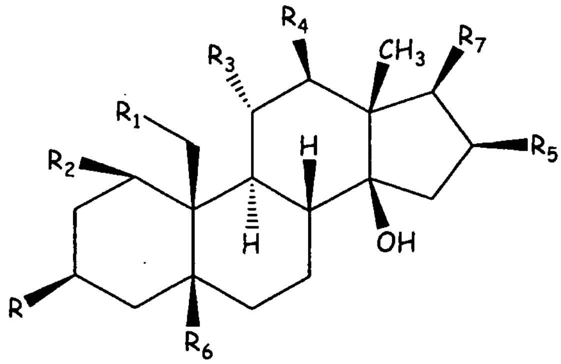

wobei
R ein Glykosid mit 1 bis 6 Zuckerresten repräsentiert;
$R_1$ Wasserstoff, -OH oder =O repräsentiert,
$R_2$, $R_3$, $R_4$, $R_5$ und $R_6$ jeweils unabhängig Wasserstoff oder-OH repräsentieren; und $R_7$ Folgendes repräsentiert

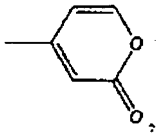

wobei das Herzglykosid einen IC50 zur Abtötung von einer oder mehreren verschiedenen Krebszelllinien von 500 nM oder weniger hat.

**8.** Kit zur Verwendung nach Anspruch 6, wobei das Herzglykosid oder die Aglykon-Form davon Proscillaridin ist.

**9.** Kit zur Verwendung nach Anspruch 6, wobei das genannte Antikrebsmittel ein Inhibitor der Chromatinfunktion, ein DNA-Topoisomerase-Inhibitor, ein Mikrotubulus-hemmendes Arzneimittel, ein DNA-schädigendes Mittel, ein Antimetabolit, ein DNA-Synthese-Inhibitor oder ein DNA-Bindemittel ist.

**10.** Kit zur Verwendung nach Anspruch 6, wobei das genannte Antikrebsmittel Gemcitabin, Mitoxanthron, Paclitaxel oder Carboplatin ist; und das genannte Herzglykosid oder die Aglykon-Form davon Proscillaridin ist.

**11.** $Na^+/K^+$-ATPase-Inhibitor in Form eines Herzglykosids oder der Aglykon-Form davon zur Verwendung in Kombination mit einem Antikrebsmittel zur Verwendung bei der Behandlung von Bauchspeicheldrüsenkrebs, **dadurch gekennzeichnet, dass** das genannte Herzglykosid oder die Aglykon-Form davon einen Steroidkern mit einem Pyron-Substituenten bei C17 umfasst (die "Bufadienoliden-Form").

**12.** Herzglykosid oder die Aglykon-Form davon zur Verwendung nach Anspruch 11, wobei das Herzglykosid oder die Aglykon-Form davon ein Herzglykosid ist, das durch die folgende allgemeine Formel repräsentiert ist:

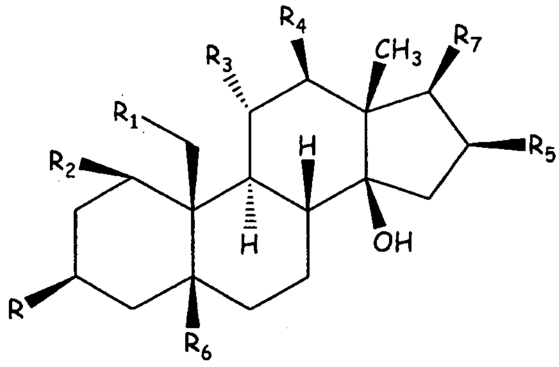

wobei
R Glykosid mit 1 bis 6 Zuckerresten repräsentiert;
$R_1$ Wasserstoff, -OH oder =O repräsentiert,
$R_2$, $R_3$, $R_4$, $R_5$ und $R_6$ jeweils unabhängig Wasserstoff oder -OH repräsentieren; und $R_7$ Folgendes repräsentiert

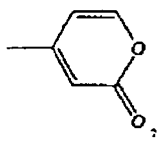

wobei das Herzglykosid einen IC50 zur Abtötung von einer oder mehreren verschiedenen Krebszelllinien von 500 nM oder weniger hat.

**13.** Herzglykosid oder die Aglykon-Form davon zur Verwendung nach Anspruch 11, wobei das Herzglykosid oder die Aglykon-Form davon Proscillaridin ist.

**14.** Herzglykosid oder die Aglykon-Form davon zur Verwendung nach Anspruch 11, wobei das genannte Antikrebsmittel ein Inhibitor der Chromatinfunktion, ein DNA-Topoisomerase-Inhibitor, ein Mikrotubulus-hemmendes Arzneimittel, ein DNA-schädigendes Mittel, ein Antimetabolit, ein DNA-Synthese-Inhibitor oder ein DNA-Bindemittel ist.

**15.** Herzglykosid oder die Aglykon-Form davon zur Verwendung nach Anspruch 11, wobei das genannte Antikrebsmittel Gemcitabin, Mitoxanthron, Paclitaxel oder Carboplatin ist; und das genannte Herzglykosid oder die Aglykon-Form davon Proscillaridin ist.

## Revendications

**1.** Formule pharmaceutique comprenant un inhibiteur de Na+/$K^+$-ATPase sous forme d'un glycoside cardiaque ou d'une forme aglycone de celui-ci, en combinaison avec un agent anticancéreux, formulé dans un excipient pharmaceutiquement admissible, pour utilisation dans le traitement du cancer du pancréas, **caractérisée en ce que** ledit glycoside cardiaque ou sa forme aglycone comprend un noyau stéroïde avec substituant de la pyrone en position C17 ( «forme bufadiénolide»).

**2.** Formule pharmaceutique pour utilisation selon la revendication 1, dans laquelle le glycoside cardiaque ou sa forme aglycone est un glycoside cardiaque représenté par la formule générale suivante :

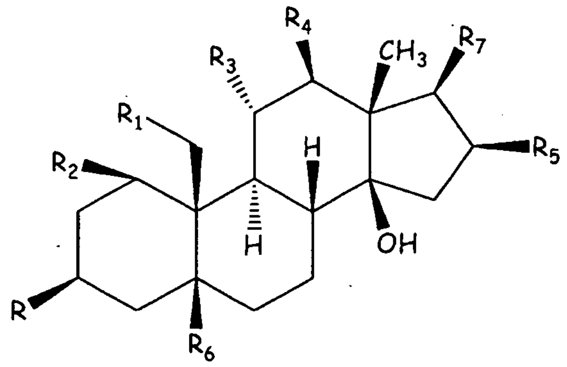

dans laquelle
R représente un glycoside de 1 à 6 résidus de sucre ;
$R_1$ représente hydrogène, -OH ou =O ;
$R_2$,$R_3$,$R_4$,$R_5$ et $R_6$ représentent chacun hydrogène ou-OH ; et $R_7$ représente

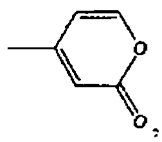

ledit glycoside cardiaque ayant une IC50 de 500nM ou moins pour tuer une ou plusieurs lignes de cellules cancéreuses différentes.

**3.** Formule pharmaceutique pour utilisation selon la revendication 1, dans laquelle le glycoside cardiaque ou sa forme aglycone est la proscillaridine.

**4.** Formule pharmaceutique pour utilisation selon la revendication 1, dans laquelle ledit agent anticancéreux est un inhibiteur des fonctions de la chromatine, un inhibiteur de la topoisomérase de l'ADN, un médicament inhibiteur de microtubules, un agent d'altération de l'ADN, un antimétabolite, un inhibiteur de la synthèse de l'ADN ou un agent de fixation à l'ADN.

**5.** Formule pharmaceutique pour utilisation selon la revendication 1, dans laquelle ledit agent anticancéreux est la gemcitabine, le mitoxantrone, le paclitaxel, or le carboplatine ; et ledit glycoside cardiaque ou sa forme aglycone est la proscillaridine.

**6.** Kit pour le traitement d'un patient atteint de cancer pancréatique, comprenant un inhibiteur de $Na^+/K^+$-ATPase sous forme d'un glycoside cardiaque ou d'une forme aglycone de celui-ci, en combinaison avec un agent anticancéreux, chacun étant formulé en doses prémesurées pour administration au patient, **caractérisé en ce que** ledit glycoside cardiaque ou sa forme aglycone comprend un noyau stéroïde avec un substituant de la pyrone en position C17 («forme bufadiénolide »).

**7.** Kit à utiliser selon la revendication 6, dans lequel le glycoside cardiaque ou une forme aglycone de celui-ci est un glycoside cardiaque représenté par la formule générale

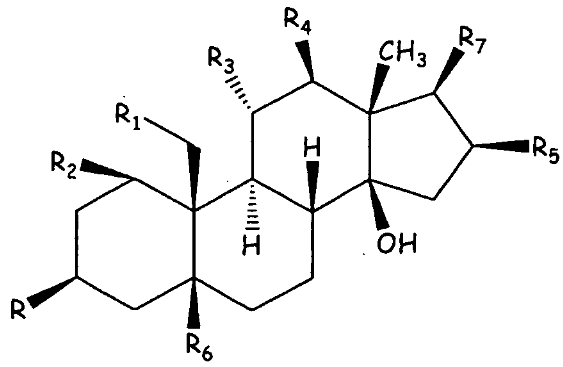

dans laquelle
R représente un glycoside de 1 à 6 résidus de sucre ;
$R_1$ représente hydrogène, —OH or =O;
$R_2$, $R_3$, $R_4$, $R_5$, and $R_6$ représentent chacun hydrogène ou—OH; et $R_7$ représente

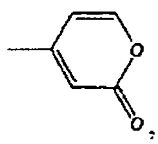

ledit glycoside cardiaque ayant une IC50 de 500 nM ou moins pour tuer une ou plusieurs lignes de cellules cancéreuses différentes.

**8.** Kit à utiliser selon la revendication 6, dans lequel le glycoside cardiaque ou sa forme aglycone est la proscillaridine.

**9.** Kit à utiliser selon la revendication 6, dans lequel ledit agent anticancéreux est un inhibiteur des fonctions de la chromatine, un inhibiteur de la topoisomérase de l'ADN, un médicament inhibiteur de microtubules, un agent d'altération de l'ADN, un antimétabolite, un inhibiteur de la synthèse de l'ADN ou un agent de fixation à l'ADN.

**10.** Kit à utiliser selon la revendication 6, dans lequel ledit agent anticancéreux est la gemcitabine, le mitoxantrone, le paclitaxel, ou la carboplatine; et ledit glycoside cardiaque ou une forme aglycone de celui-ci est la proscillaridine.

**11.** Inhibiteur de Na+/K+-TPase sous forme d'un glycoside cardiaque ou d'une forme aglycone de celui-ci, en combinaison avec un agent anticancéreux, pour utilisation dans le traitement du cancer pancréatique, **caractérisé en ce que** ledit glycoside cardiaque ou une forme aglycone de celui-ci comprend un noyau stéroïde avec un substituant de la pyrone en position C17 («forme bufadiénolide»).

**12.** Glycoside cardiaque ou forme aglycone de celui-ci, pour utilisation selon la revendication 11, dans lequel le glycoside cardiaque ou forme aglycone de celui-ci est un glycoside cardiaque représenté par la formule générale:

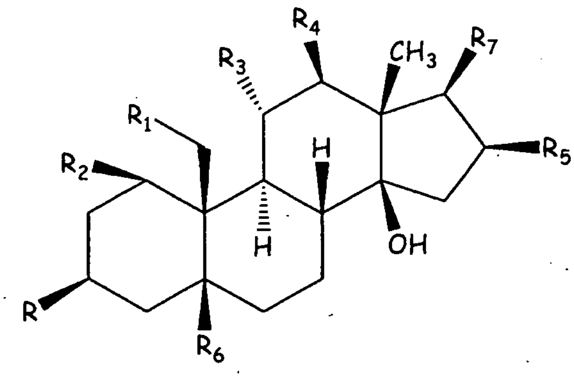

dans laquelle
R représente un glycoside de 1 à 6 résidus de sucre ;
$R_1$ représente hydrogène, -OH or =O;
$R_2$, $R_3$, $R_4$, $R_5$, and $R_6$ représentent chacun hydrogène ou—OH; and $R_7$ représente

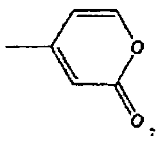

ledit glycoside cardiaque ayant une IC50 de 500nM ou moins, pour tuer une ou plusieurs lignes de cellules cancéreuses différentes.

**13.** Glycoside cardiaque ou forme aglycone de celui-ci pour utilisation selon la revendication 11, dans lequel le glycoside cardiaque ou sa forme aglycone est la proscillaridine.

**14.** Glycoside cardiaque ou forme aglycone de celui-ci pour utilisation selon la revendication 11, dans lequel ledit agent anticancéreux est un inhibiteur des fonctions de la chromatine, un inhibiteur de la topoisomérase de l'ADN, un médicament inhibiteur de microtubules, an agent d'altération de l'ADN, un antimétabolite, un inhibiteur de la synthèse de l'ADN ou un agent de fixation à l'ADN.

**15.** Glycoside cardiaque ou forme aglycone de celui-ci pour utilisation selon la revendication 11, dans lequel ledit agent anticancéreux est la gemcitabine, le mitoxantrone, le paclitaxel ou le carboplatine ; et ledit glycoside cardiaque ou forme aglycone de celui-ci est la proscillaridine.

Figure 1

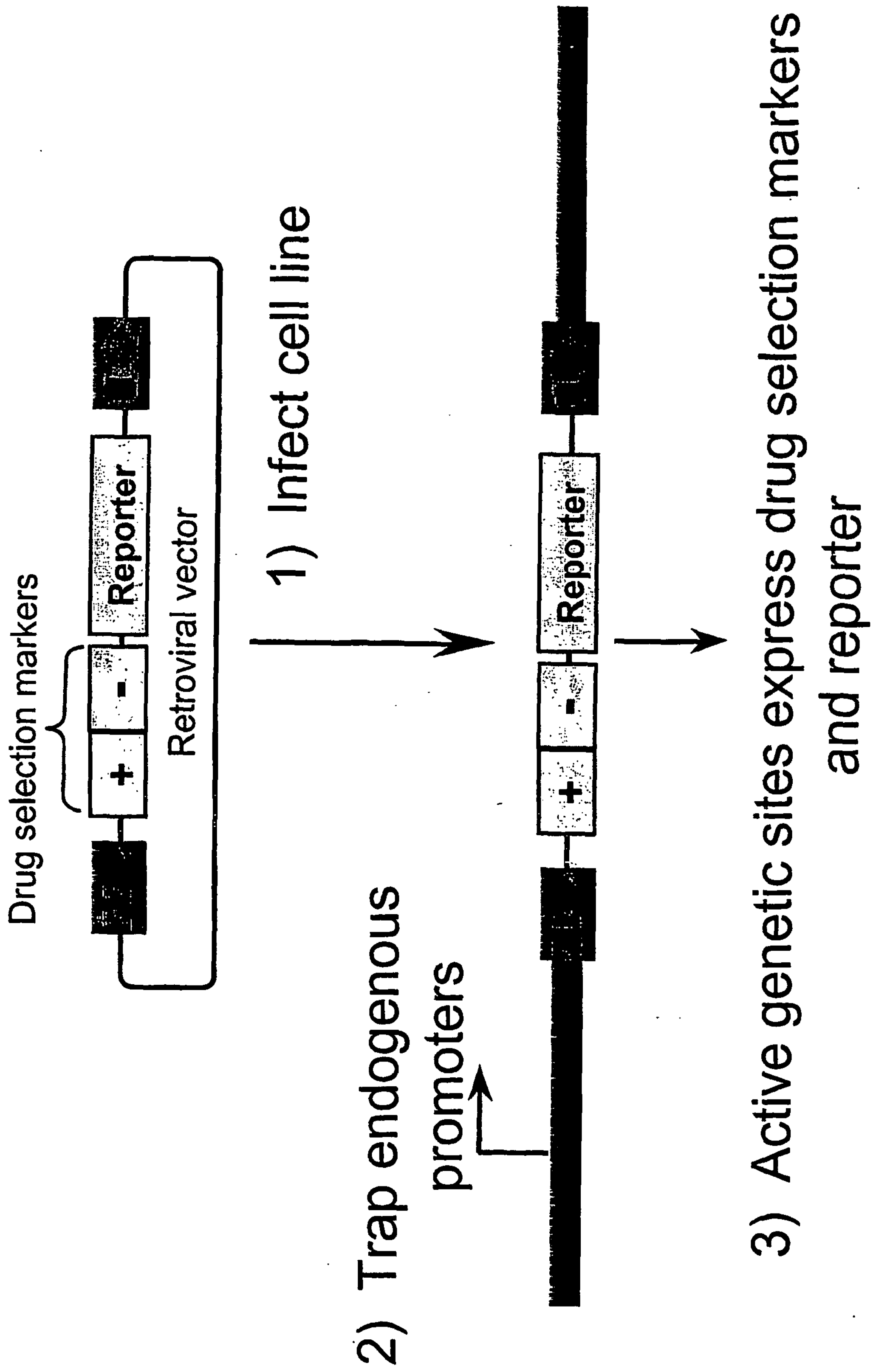
Drug selection markers
+
-
Reporter
Retroviral vector
1) Infect cell line
2) Trap endogenous promoters
+
-
Reporter
3) Active genetic sites express drug selection markers and reporter

Figure 2

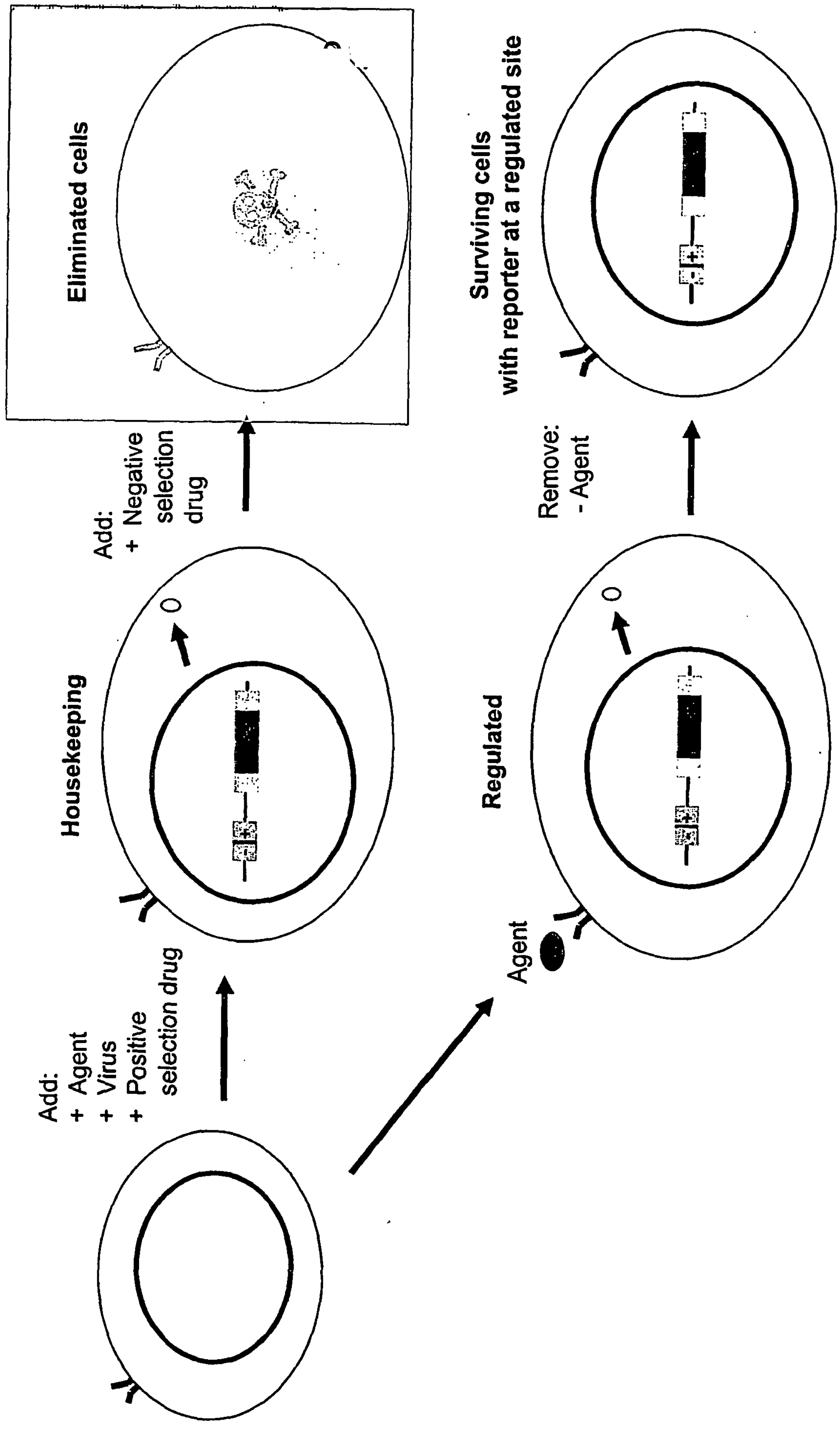
Add:
+ Agent
+ Virus
+ Positive selection drug
Housekeeping
Add:
+ Negative selection drug
Eliminated cells
Agent
Regulated
Remove:
- Agent
Surviving cells
with reporter at a regulated site

# Figure 3

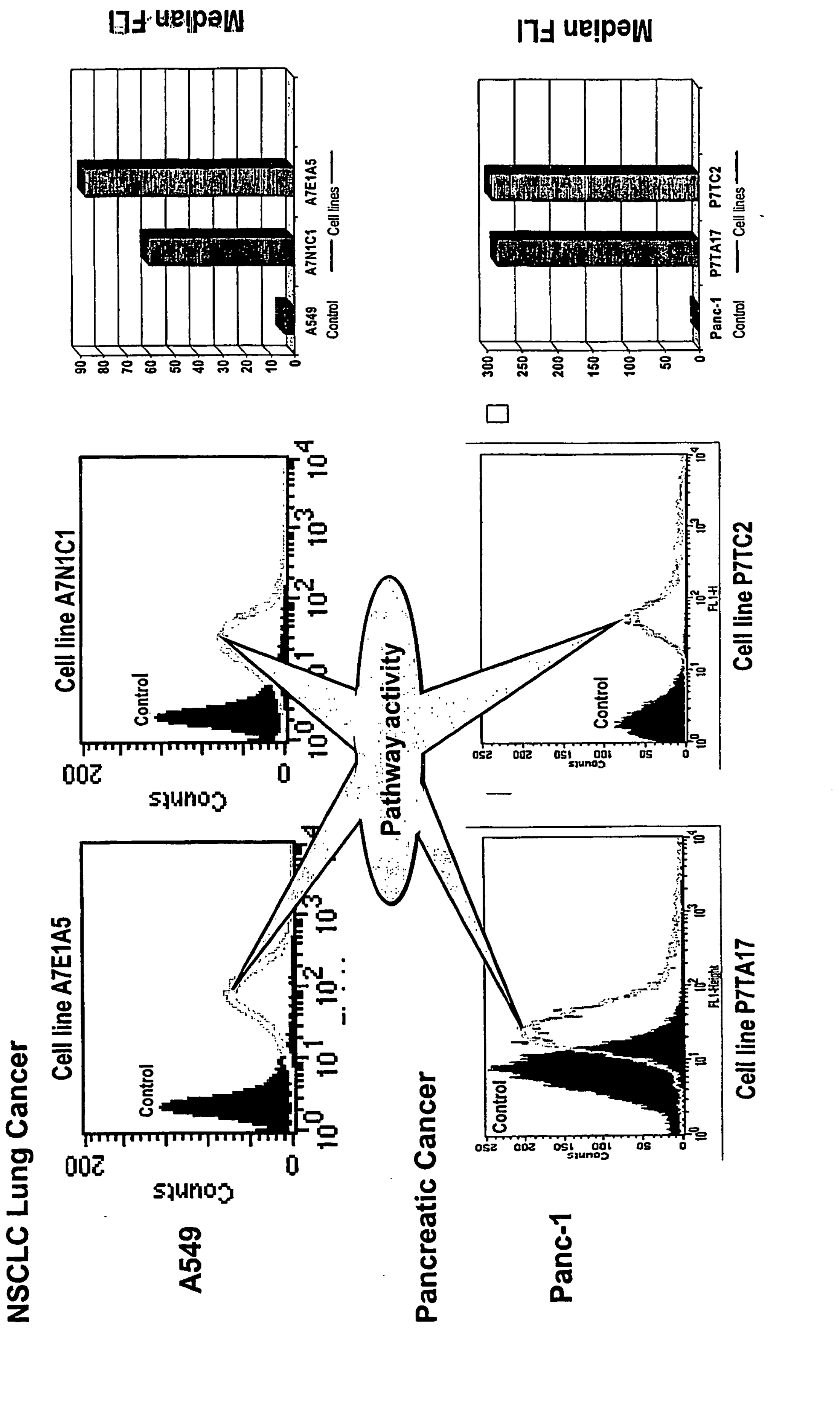
NSCLC Lung Cancer
A549
Cell line A7E1A5
Cell line A7N1C1
Control
Counts
200
0
Pathway activity
Median FLI
A549 Control
A7N1C1
A7E1A5
Cell lines
Pancreatic Cancer
Panc-1
Cell line P7TA17
Cell line P7TC2
Panc-1 Control
P7TA17
P7TC2

Figure 4

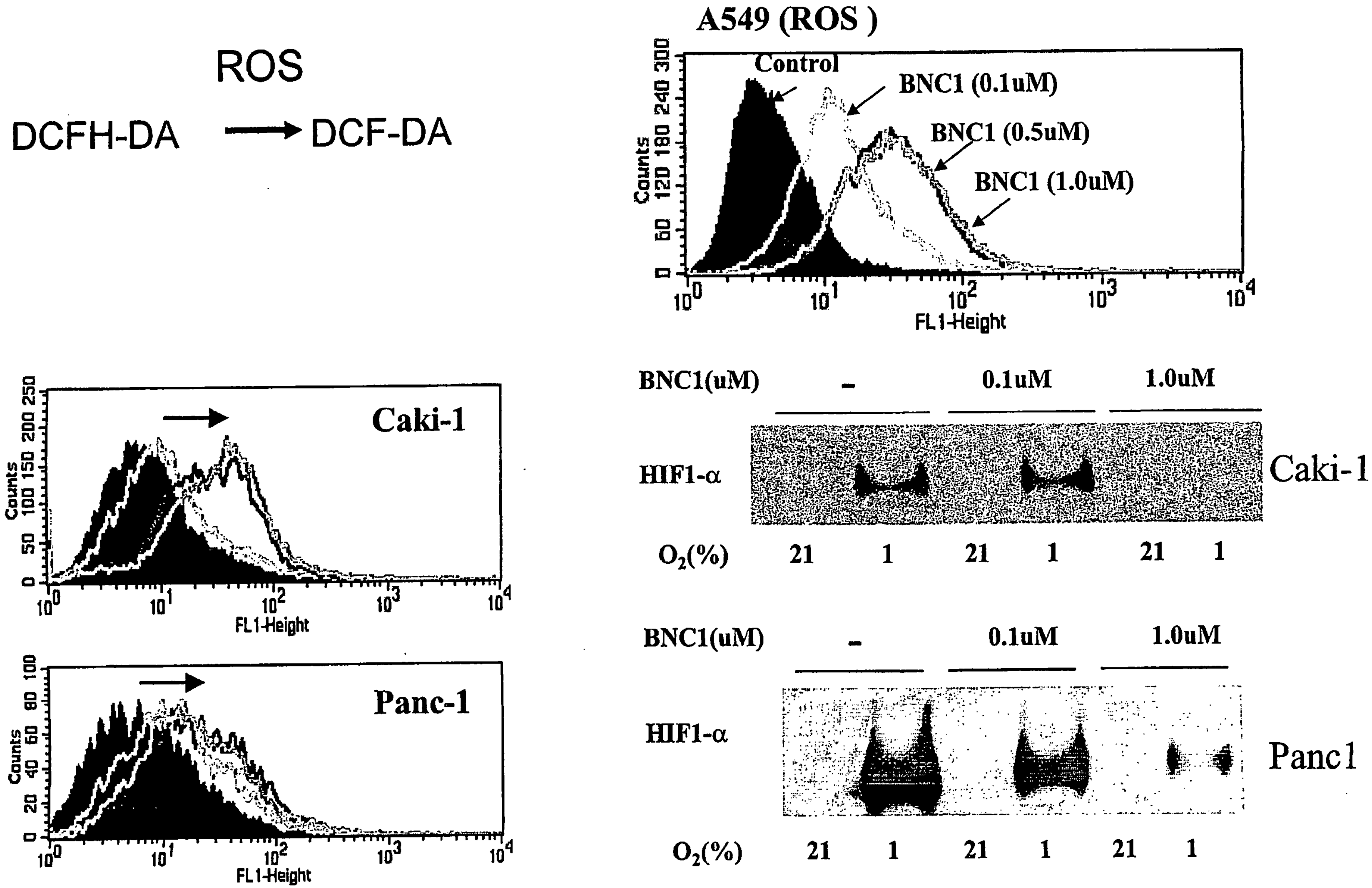
ROS
DCFH-DA → DCF-DA
A549 (ROS )
Control
BNC1 (0.1uM)
BNC1 (0.5uM)
BNC1 (1.0uM)
Counts
FL1-Height
Caki-1
Panc-1
BNC1(uM)
-
0.1uM
1.0uM
HIF1-α
Caki-1
$O_2$(%)
21
1
21
1
21
1
Panc1

Figure 5

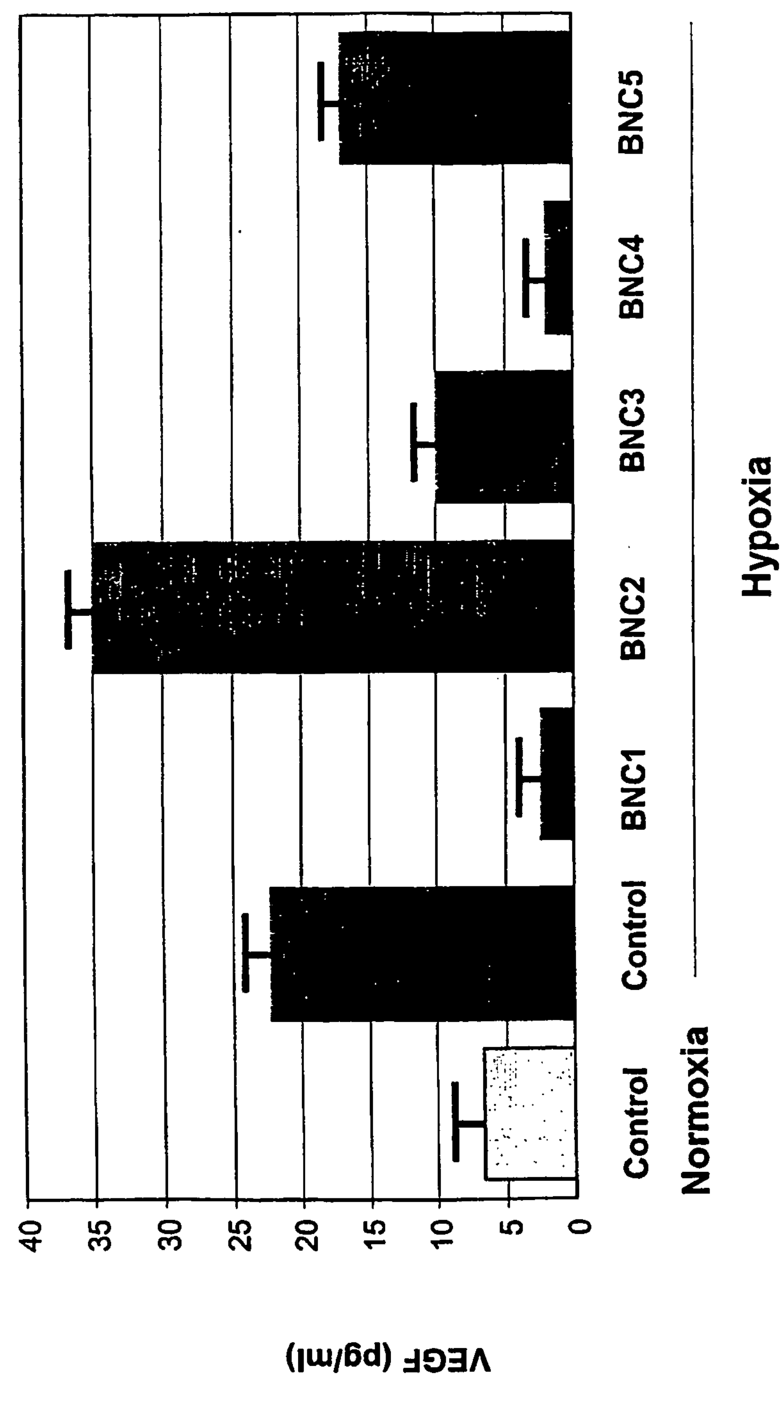

VEGF (pg/ml)
0
5
10
15
20
25
30
35
40
Control
Control
BNC1
BNC2
BNC3
BNC4
BNC5
Normoxia
Hypoxia

Figure 6

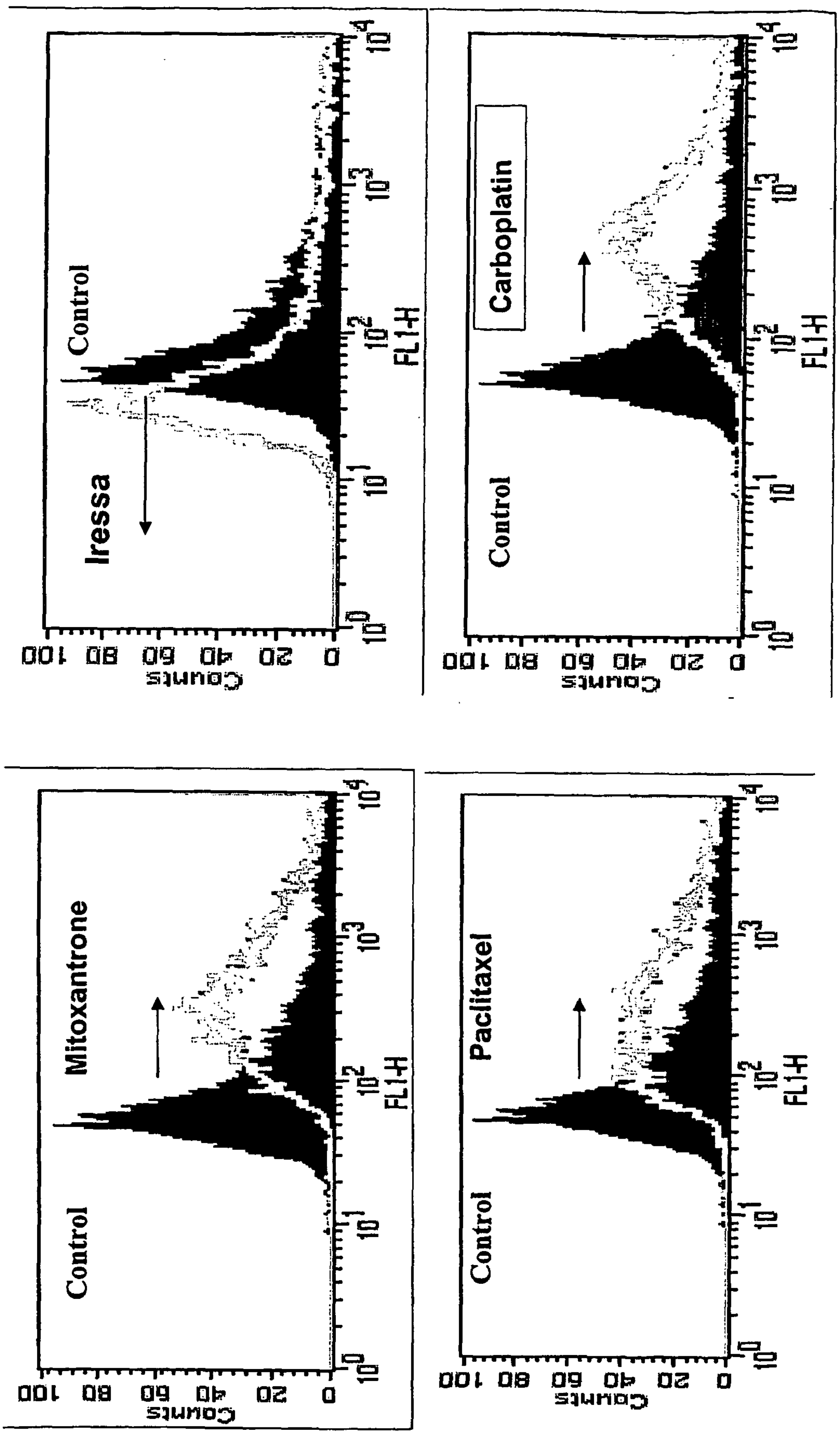
Control
Iressa
Control
Carboplatin
Control
Mitoxantrone
Control
Paclitaxel
FL1-H
Counts

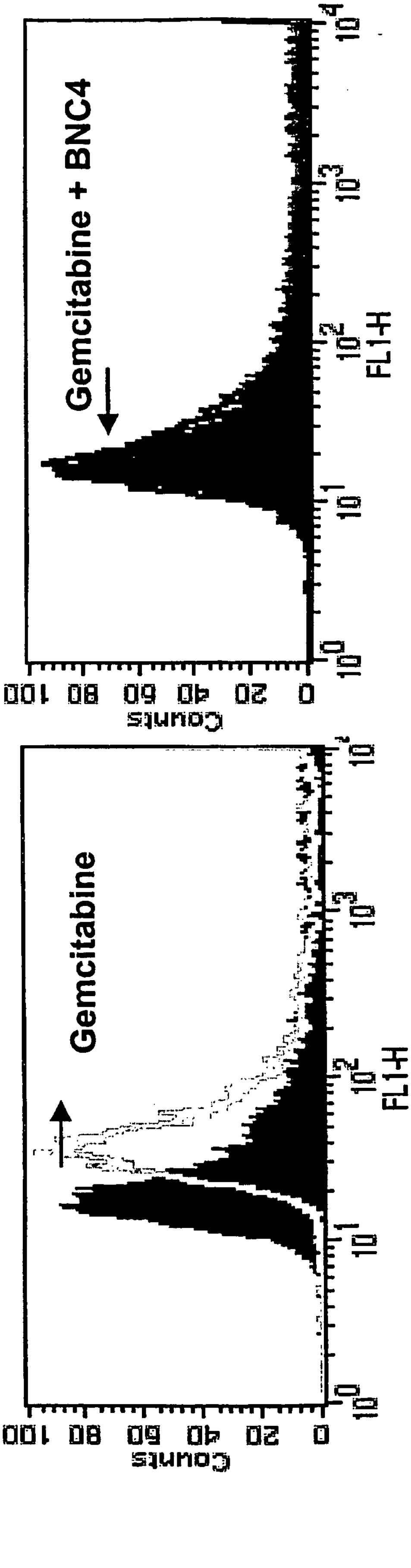


Figure 7

Figure 8

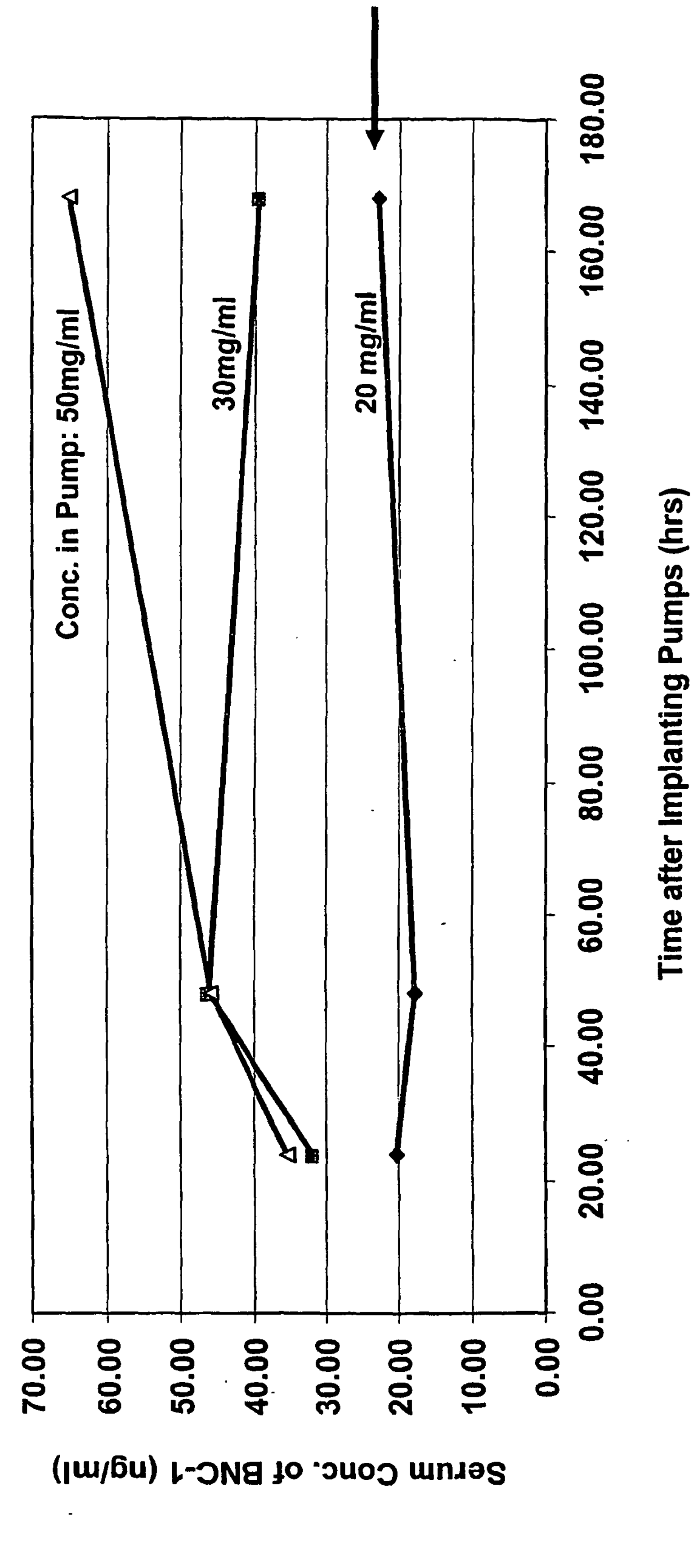
Serum Conc. of BNC-1 (ng/ml)
70.00
60.00
50.00
40.00
30.00
20.00
10.00
0.00
Time after Implanting Pumps (hrs)
0.00
20.00
40.00
60.00
80.00
100.00
120.00
140.00
160.00
180.00
Conc. in Pump: 50mg/ml
30mg/ml
20 mg/ml

**Figure 9**

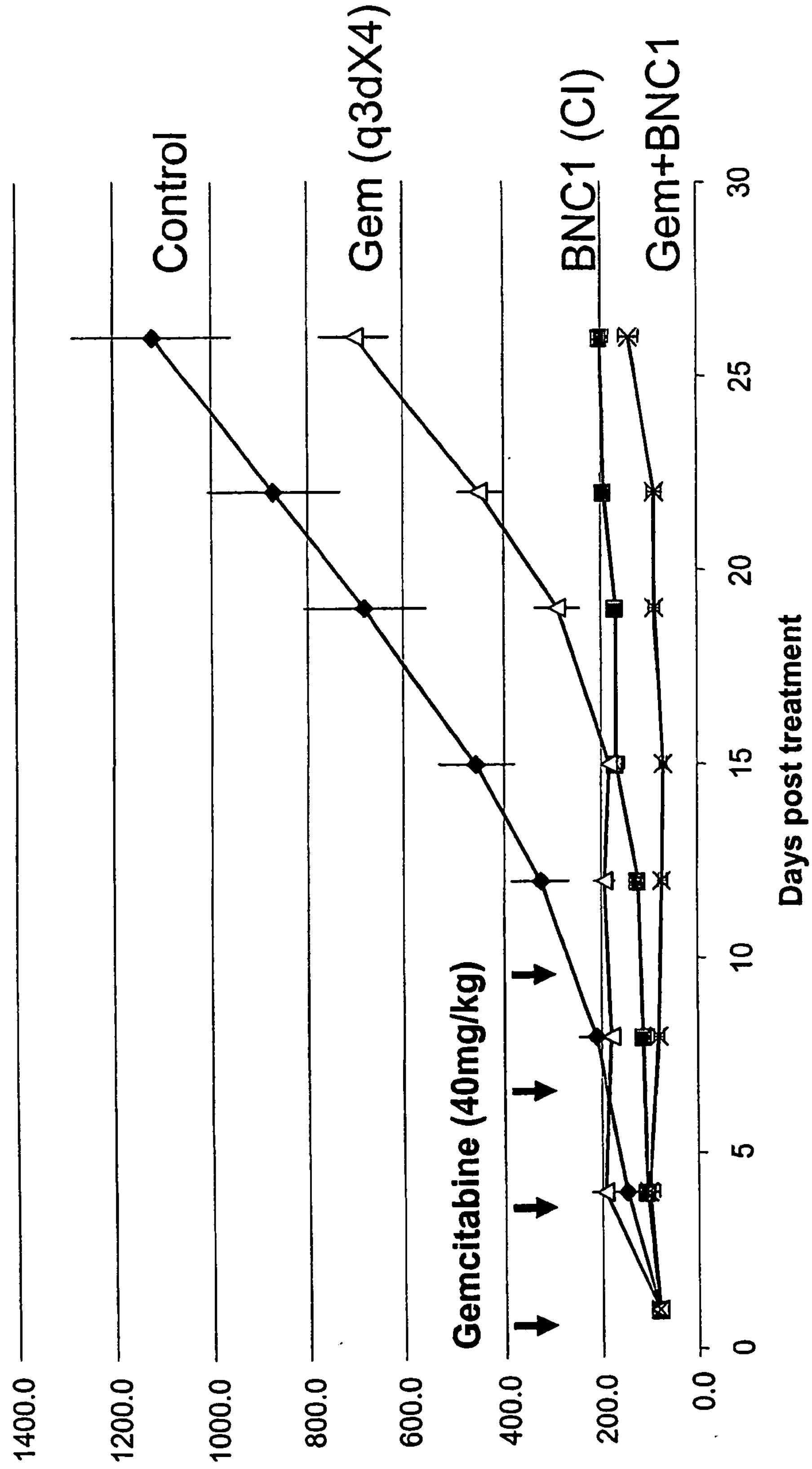
Tumor Weight (mg)
1400.0
1200.0
1000.0
800.0
600.0
400.0
200.0
0.0
0
5
10
15
20
25
30
Days post treatment
Gemcitabine (40mg/kg)
Control
Gem (q3dX4)
BNC1 (CI)
Gem+BNC1

Figure 10

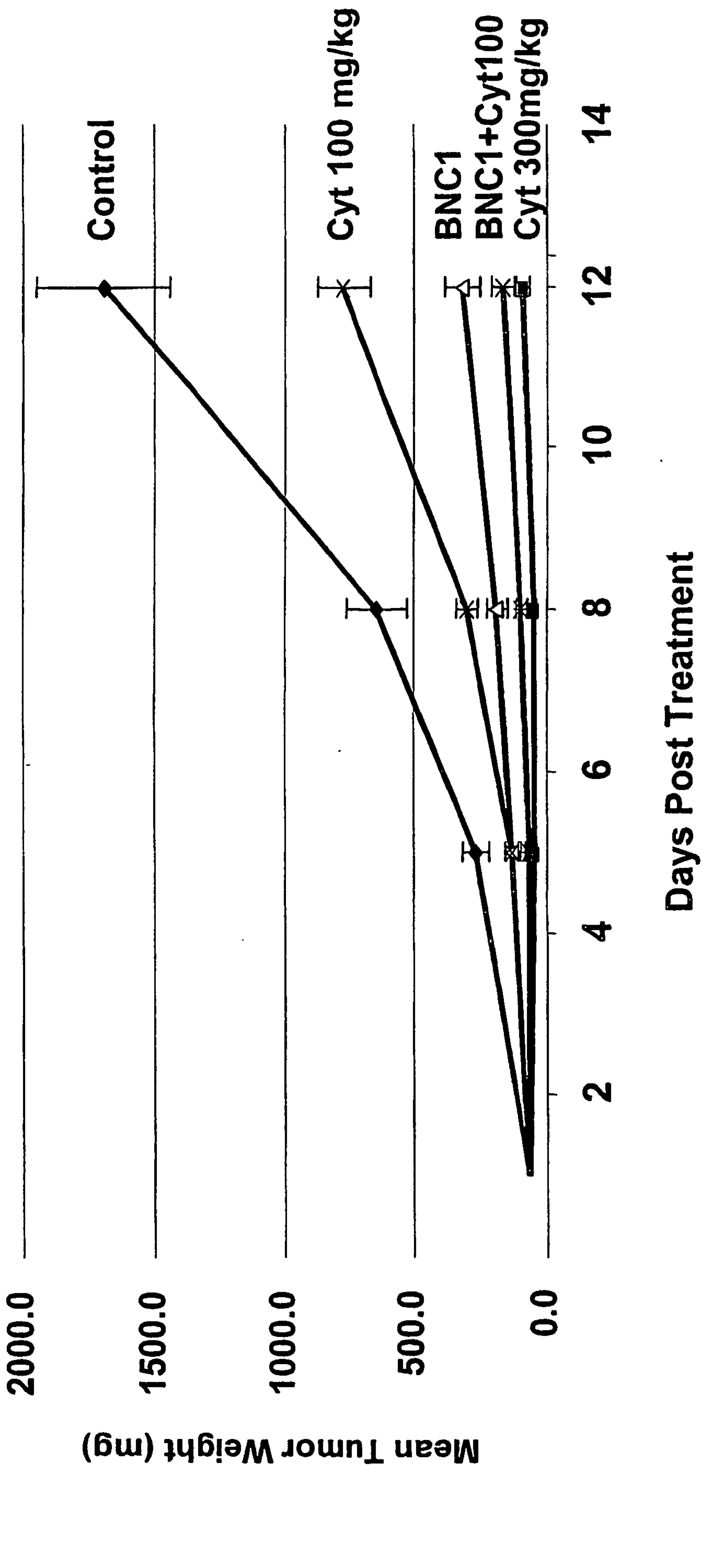
Control
Cyt 100 mg/kg
BNC1
BNC1+Cyt100
Cyt 300mg/kg
2000.0
1500.0
1000.0
500.0
0.0
Mean Tumor Weight (mg)
2
4
6
8
10
12
14
Days Post Treatment

Figure 11

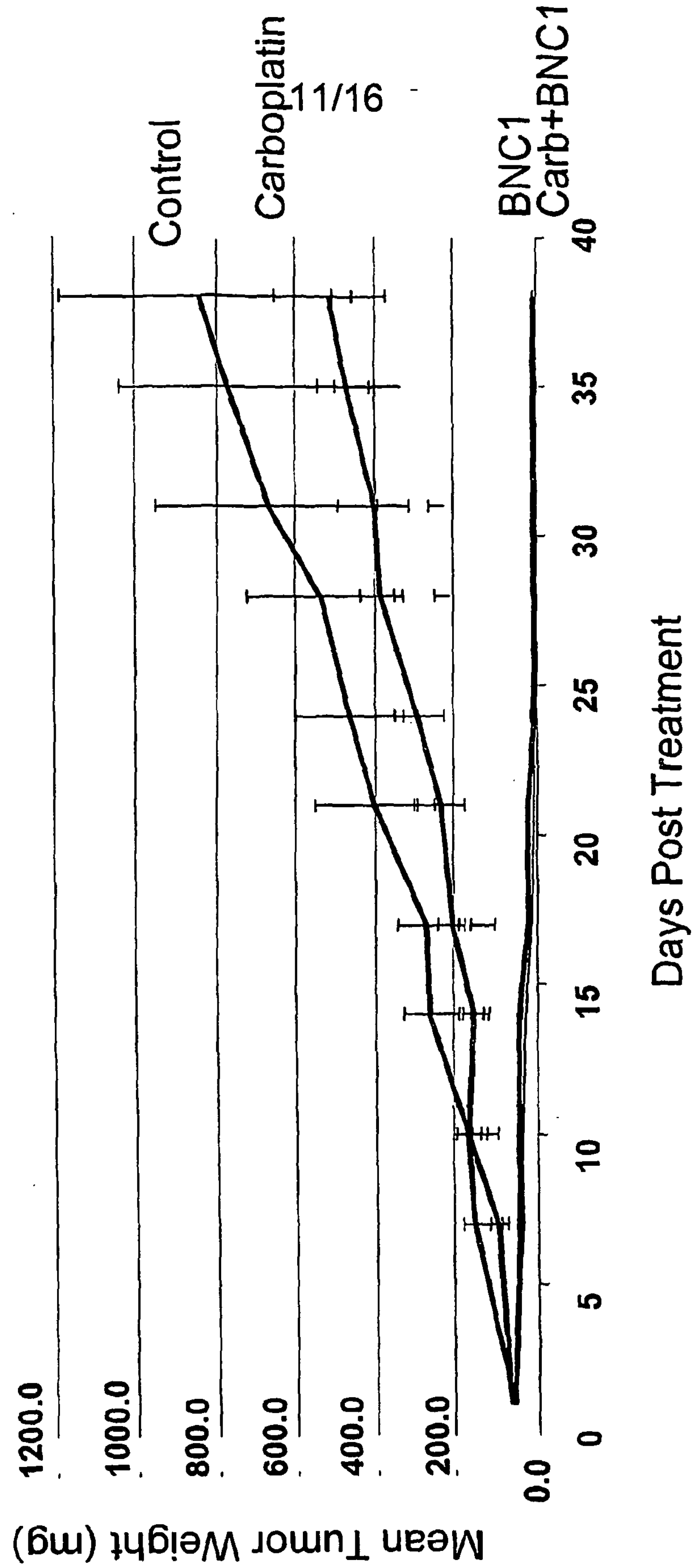
11/16
Mean Tumor Weight (mg)
1200.0
1000.0
800.0
600.0
400.0
200.0
0.0
0
5
10
15
20
25
30
35
40
Days Post Treatment
Control
Carboplatin
BNC1
Carb+BNC1

Figure 12

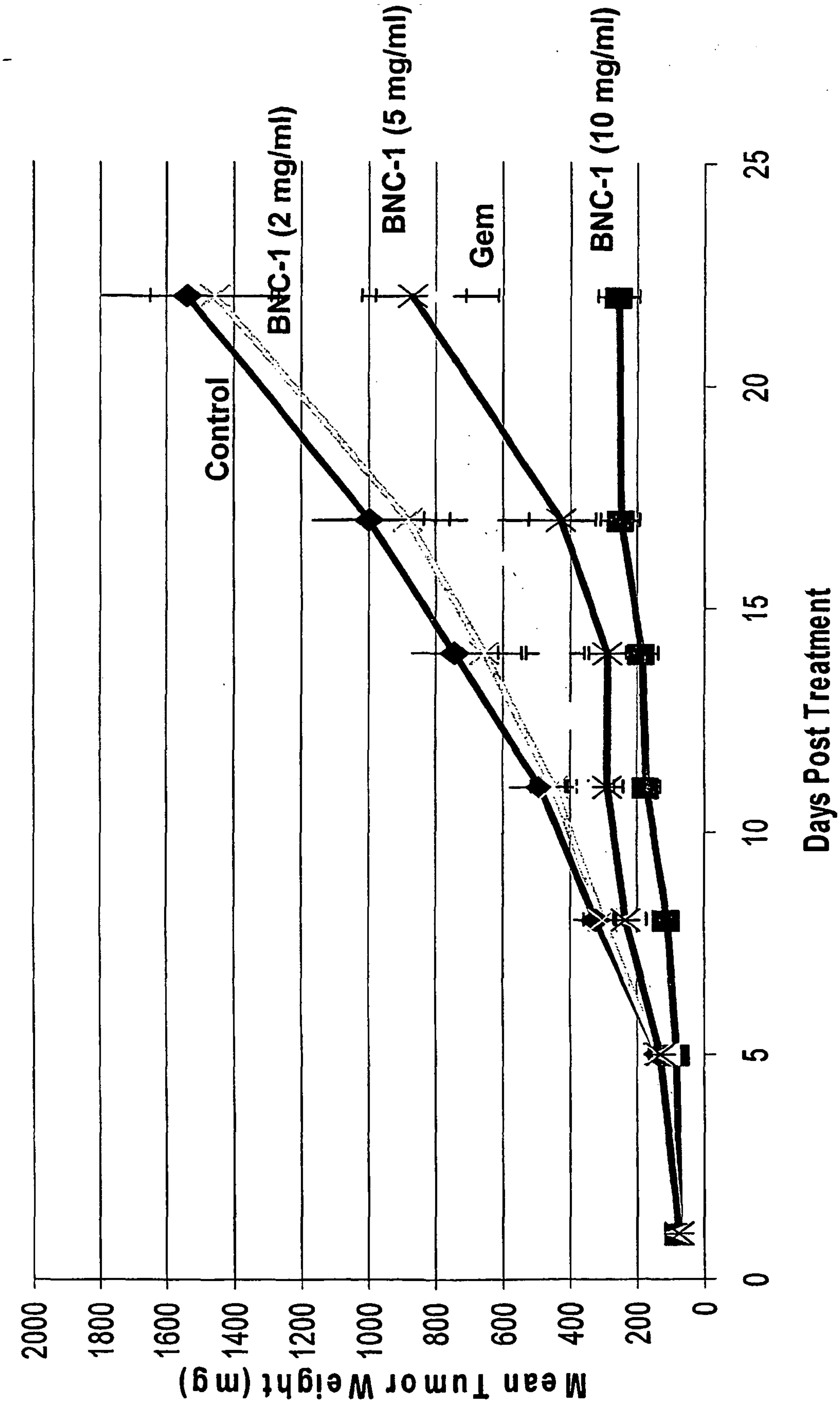
Mean Tumor Weight (mg)
2000
1800
1600
1400
1200
1000
800
600
400
200
0
0
5
10
15
20
25
Days Post Treatment
Control
BNC-1 (2 mg/ml)
BNC-1 (5 mg/ml)
Gem
BNC-1 (10 mg/ml)

Figure 13

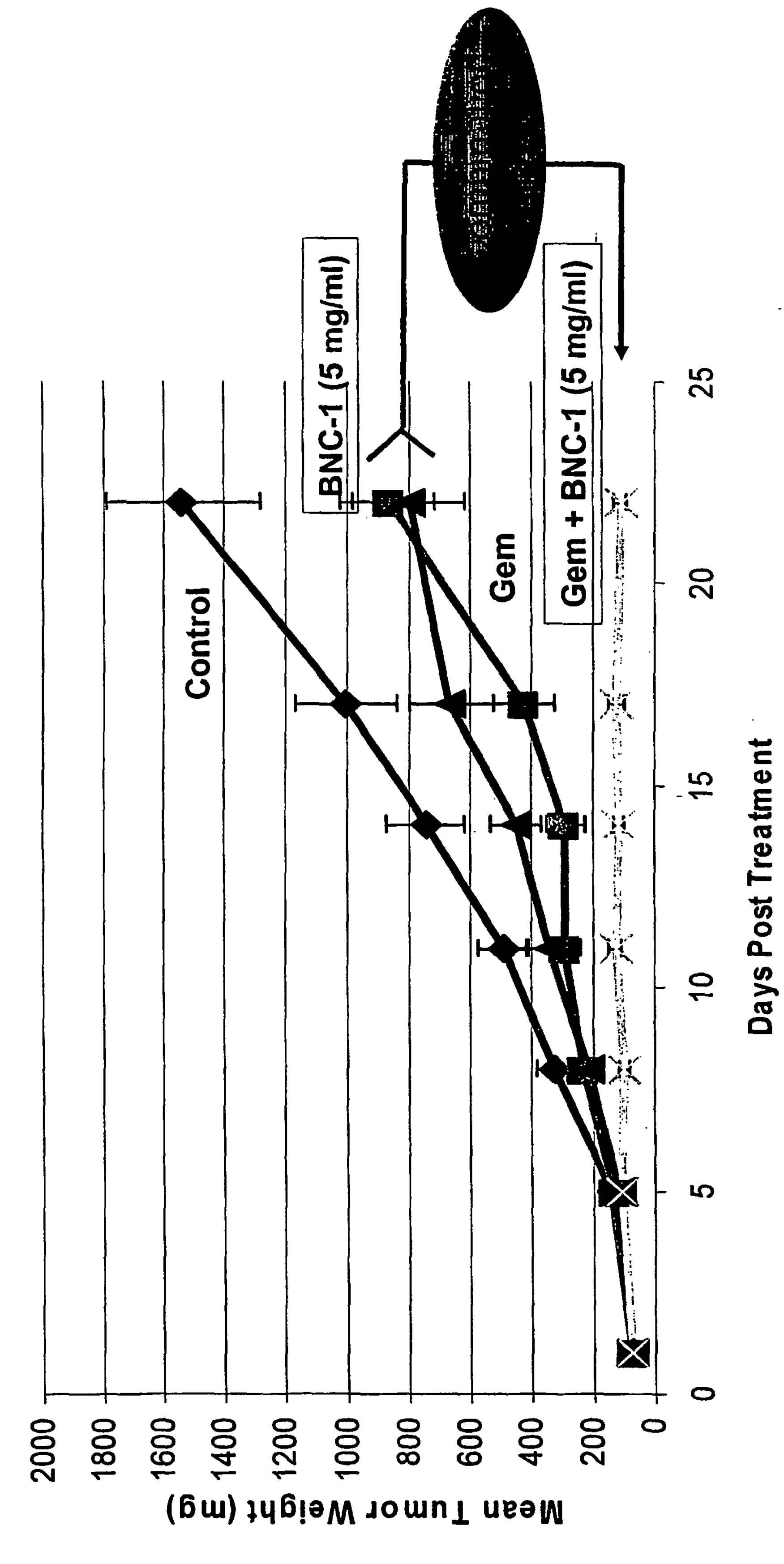

Mean Tumor Weight (mg)
2000
1800
1600
1400
1200
1000
800
600
400
200
0
0
5
10
15
20
25
Days Post Treatment
Control
Gem
BNC-1 (5 mg/ml)
Gem + BNC-1 (5 mg/ml)

Figure 14

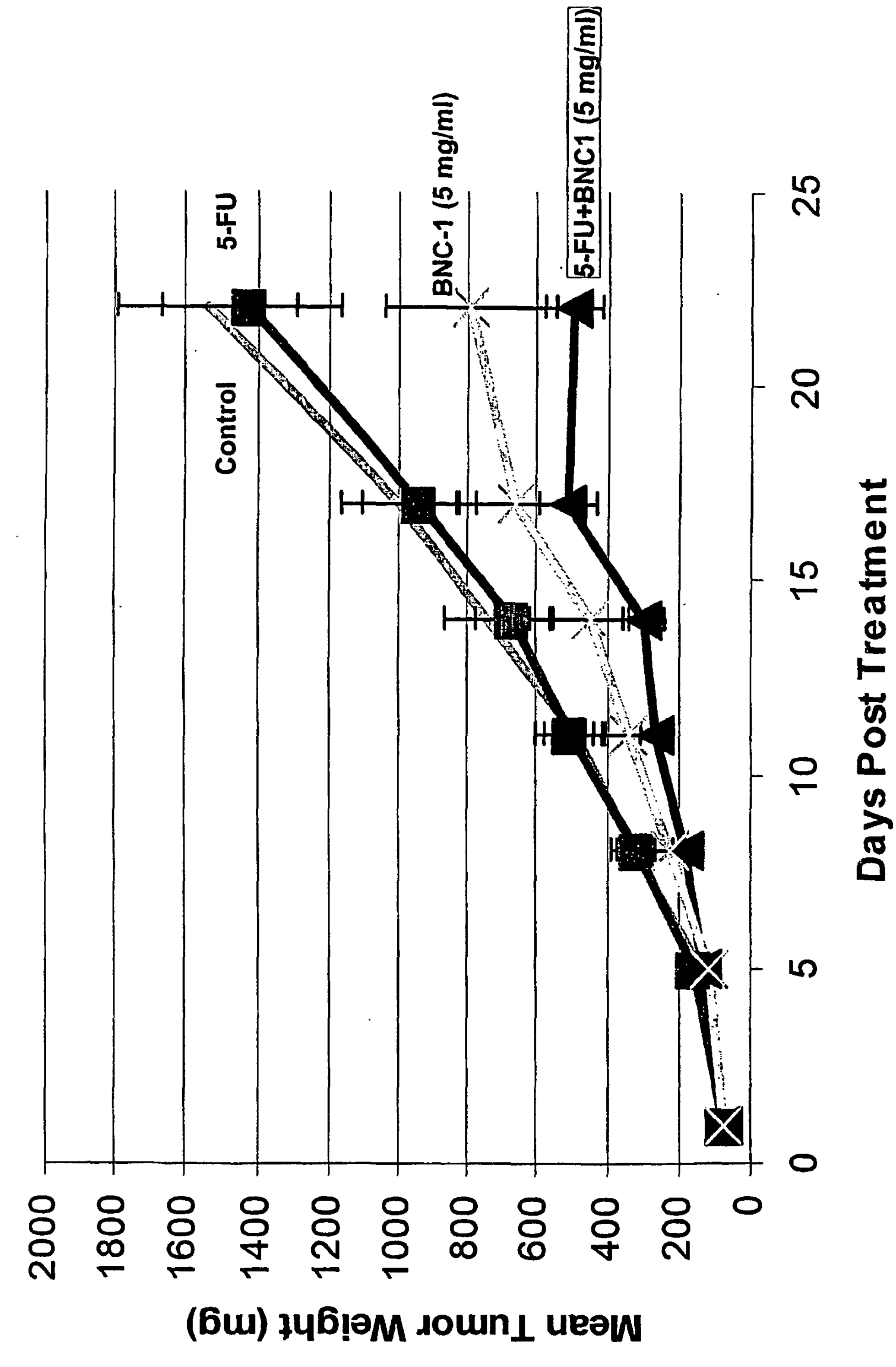
Mean Tumor Weight (mg)
2000
1800
1600
1400
1200
1000
800
600
400
200
0
Days Post Treatment
0
5
10
15
20
25
Control
5-FU
BNC-1 (5 mg/ml)
5-FU+BNC1 (5 mg/ml)

Figure 15

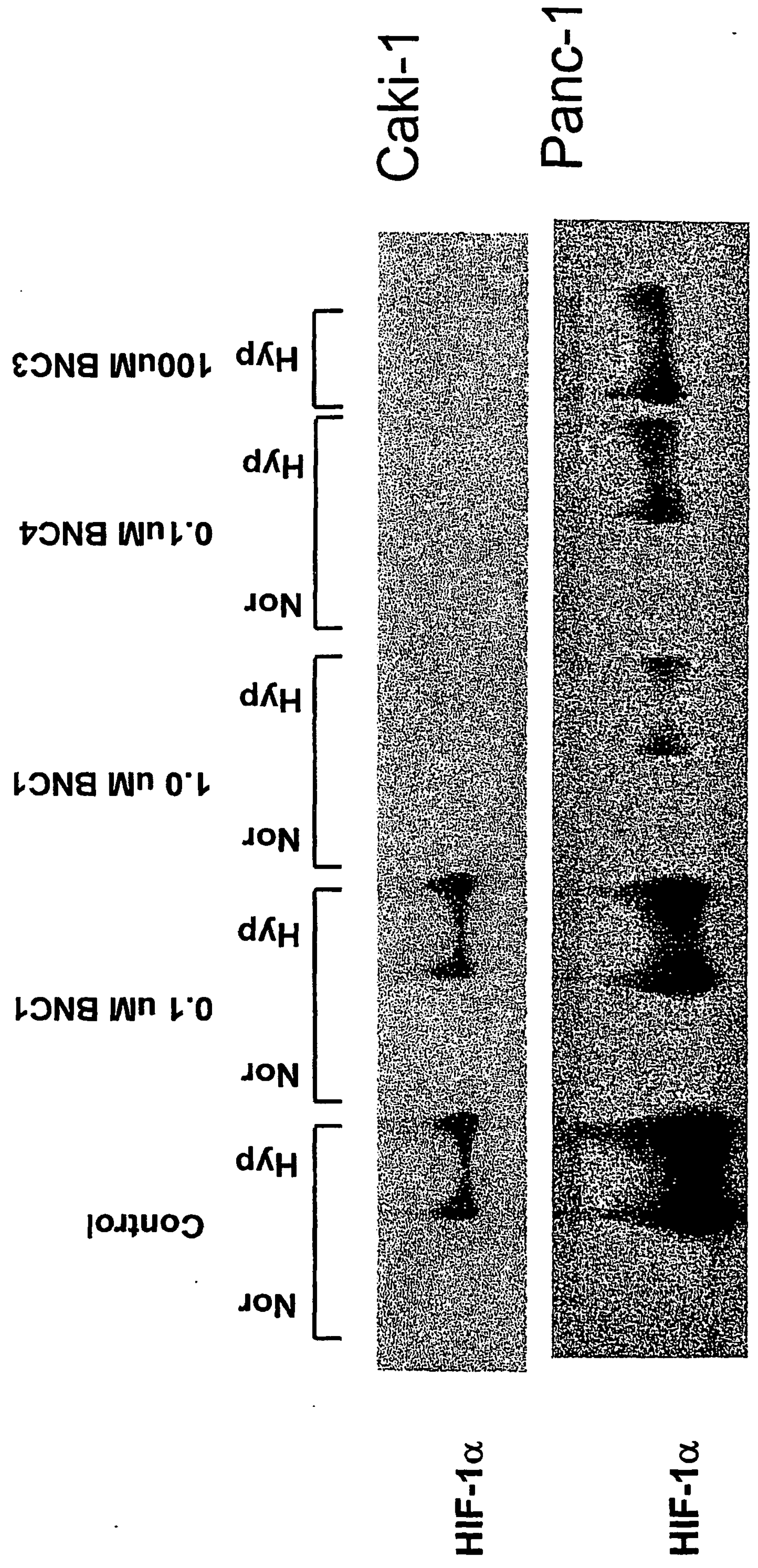
Caki-1
Panc-1
Control
0.1 uM BNC1
1.0 uM BNC1
0.1uM BNC4
100uM BNC3
Nor
Hyp
HIF-1α
HIF-1α

Figure 16

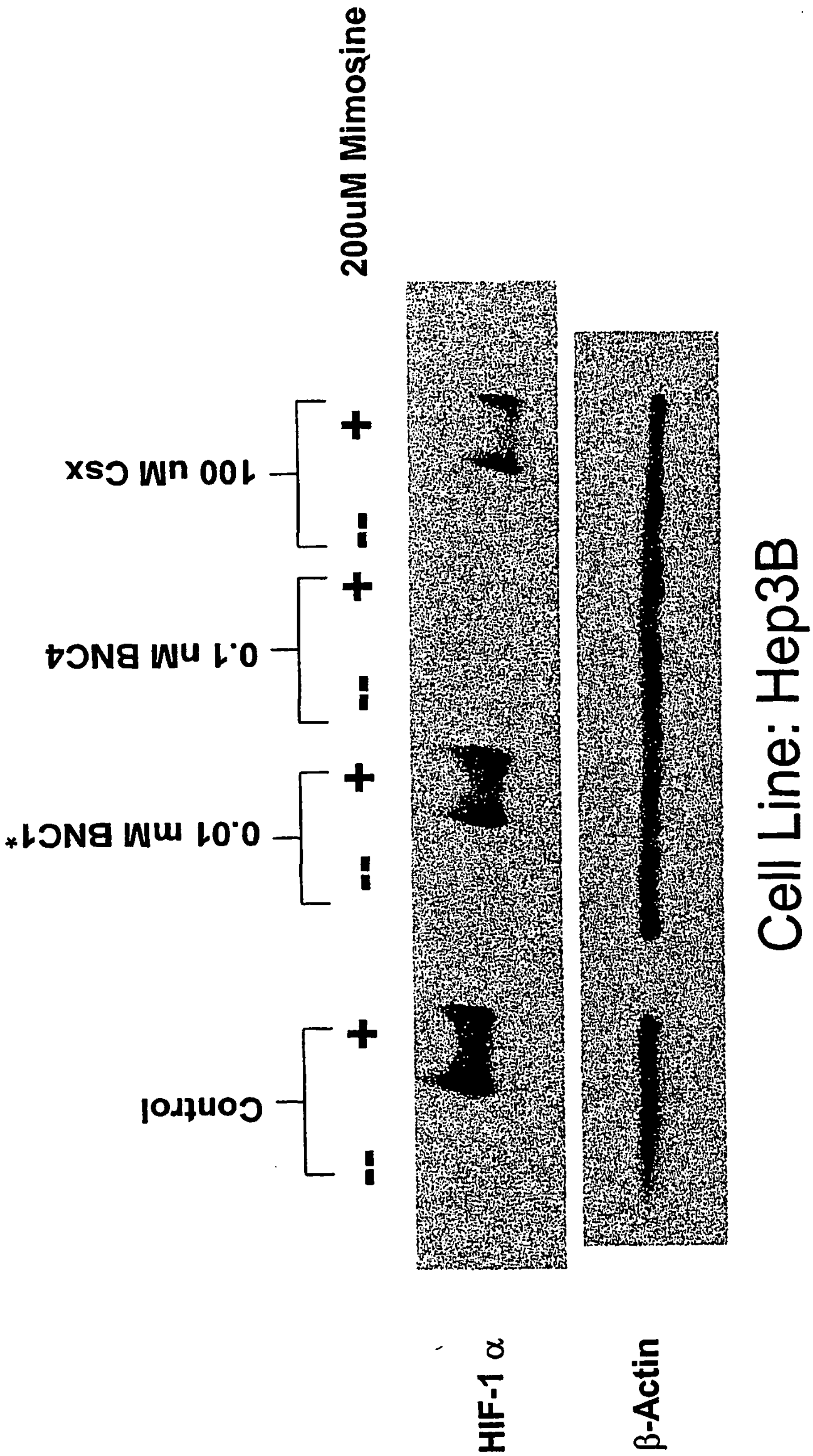
200uM Mimosine
Control
0.01 mM BNC1*
0.1 nM BNC4
100 uM Csx
-
+
-
+
-
+
-
+
HIF-1 α
β-Actin
Cell Line: Hep3B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5240714 A
- WO 0044931 A
- WO 0242842 A


### Non-patent literature cited in the description


- **Dr. John Beard.** *The Enzyme Theory of Cancer,* 1911
- **Saruc et al.** *Pancreas,* May 2004, vol. 28 (4), 401-12